# EUROPEAN PATENT SPECIFICATION

(11) **EP 2 756 081 B1**
(45) Date of publication and mention of the grant of the patent: **01.07.2015**
(21) Application number: 12761794.2
(22) Date of filing: 14.09.2012
(51) Int. Cl.: C12N 15/62

(54) **THERMOSTABLE ASSAY REAGENTS**
THERMOSTABILE TESTREAGENZIEN
RÉACTIFS DE DOSAGE THERMOSTABLES

(30) Priority: 14.09.2011 GB 201115911
(43) Date of publication of application: 23.07.2014
(73) Proprietor: The Secretary of State for Health, London SW1A 2NS (GB)
(72) Inventor: SUTTON, John Mark, Salisbury Wiltshire SP4 0JG (GB); SKIPPER, Philip James Alister, Lincolnshire LN2 4DY (GB)
(74) Representative: Griffin, Philippa Jane
(86) International application number: PCT/GB2012/052281
(87) International publication number: WO 2013/038203

(56) References cited:
- DOOLEY HELEN ET AL: "Selection and characterization of naturally occurring single-domain (IgNAR) antibody fragments from immunized sharks by phage display.", September 2003 (2003-09), MOLECULAR IMMUNOLOGY, VOL. 40, NR. 1, PAGE(S) 25-33, XP003013541, ISSN: 0161-5890 page 27, right-hand column, paragraph 2 page 30, left-hand column, paragraph 2 - paragraph 5
- GHAHROUDI M ARBABI ET AL: "Selection and identification of single domain antibody fragments from camel heavy-chain antibodies", 1997, FEBS LETTERS, VOL. 414, NR. 3, PAGE(S) 521-526, XP002688154, ISSN: 0014-5793 page 524, left-hand column, paragraph 2 - right-hand column, paragraph 3

### SEQUENCE LISTING

<110> Health Protection Agency
<120> Thermostable assay reagents
<130> P36821WO-PJG
<150> GB1115911.8
   <151> 2011-09-14
<160> 21
<170> PatentIn version 3.5
<210> 1
   <211> 408
   <212> PRT
   <213> Methanosarcina thermophila
<400> 1
   Met Lys Val Leu Val Ile Asn Ala Gly Ser Ser Ser Leu Lys Tyr Gln
   1               5                   10                  15
   Leu Ile Asp Met Thr Asn Glu Ser Ala Leu Ala Val Gly Leu Cys Glu
               20                  25                  30
   Arg Ile Gly Ile Asp Asn Ser Ile Ile Thr Gln Lys Lys Phe Asp Gly
           35                  40                  45
   Lys Lys Leu Glu Lys Leu Thr Asp Leu Pro Thr His Lys Asp Ala Leu
       50                  55                  60
   Glu Glu Val Val Lys Ala Leu Thr Asp Asp Glu Phe Gly Val Ile Lys
   65                  70                  75                  80
   Asp Met Gly Glu Ile Asn Ala Val Gly His Arg Val Val His Gly Gly
                   85                  90                  95
   Glu Lys Phe Thr Thr Ser Ala Leu Tyr Asp Glu Gly Val Glu Lys Ala
               100                 105                 110
   Ile Lys Asp Cys Phe Glu Leu Ala Pro Leu His Asn Pro Pro Asn Met
           115                 120                 125
   Met Gly Ile Ser Ala Cys Ala Glu Ile Met Pro Gly Thr Pro Met Val
       130                 135                 140
   Ile Val Phe Asp Thr Ala Phe His Gln Thr Met Pro Pro Tyr Ala Tyr
   145                 150                 155                 160
   Met Tyr Ala Leu Pro Tyr Asp Leu Tyr Glu Lys His Gly Val Arg Lys
   165 170 175
   Tyr Gly Phe His Gly Thr Ser His Lys Tyr Val Ala Glu Arg Ala Ala
   180 185 190
   Leu Met Leu Gly Lys Pro Ala Glu Glu Thr Lys Ile Ile Thr Cys His
   195 200 205
   Leu Gly Asn Gly Ser Ser Ile Thr Ala Val Glu Gly Gly Lys Ser Val
   210 215 220
   Glu Thr Ser Met Gly Phe Thr Pro Leu Glu Gly Leu Ala Met Gly Thr
   225 230 235 240
   Arg Cys Gly Ser Ile Asp Pro Ala Ile Val Pro Phe Leu Met Glu Lys
   245 250 255
   Glu Gly Leu Thr Thr Arg Glu Ile Asp Thr Leu Met Asn Lys Lys Ser
   260 265 270
   Gly Val Leu Gly Val Ser Gly Leu Ser Asn Asp Phe Arg Asp Leu Asp
   275 280 285
   Glu Ala Ala Ser Lys Gly Asn Arg Lys Ala Glu Leu Ala Leu Glu Ile
   290 295 300
   Phe Ala Tyr Lys Val Lys Lys Phe Ile Gly Glu Tyr Ser Ala Val Leu
   305 310 315 320
   Asn Gly Ala Asp Ala Val Val Phe Thr Ala Gly Ile Gly Glu Asn Ser
   325 330 335
   Ala Ser Ile Arg Lys Arg Ile Leu Thr Gly Leu Asp Gly Ile Gly Ile
   340 345 350
   Lys Ile Asp Asp Glu Lys Asn Lys Ile Arg Gly Gln Glu Ile Asp Ile
   355 360 365
   Ser Thr Pro Asp Ala Lys Val Arg Val Phe Val Ile Pro Thr Asn Glu
   370 375 380
   Glu Leu Ala Ile Ala Arg Glu Thr Lys Glu Ile Val Glu Thr Glu Val
   385 390 395 400
   Lys Leu Arg Ser Ser Ile Pro Val
   405
<210> 2
   <211> 587
   <212> PRT
   <213> Geobacillus stearothermophilus
<400> 2
   Met Lys Arg Lys Thr Lys Ile Val Cys Thr Ile Gly Pro Ala Ser Glu
   1               5                   10                  15
   Ser Val Asp Lys Leu Val Gln Leu Met Glu Ala Gly Met Asn Val Ala
               20                  25                  30
   Arg Leu Asn Phe Ser His Gly Asp His Glu Glu His Gly Arg Arg Ile
           35                  40                  45
   Ala Asn Ile Arg Glu Ala Ala Lys Arg Thr Gly Arg Thr Val Ala Ile
       50                  55                  60
   Leu Leu Asp Thr Lys Gly Pro Glu Ile Arg Thr His Asn Met Glu Asn
   65                  70                  75                  80
   Gly Ala Ile Glu Leu Lys Glu Gly Ser Lys Leu Val Ile Ser Met Ser
                   85                  90                  95
   Glu Val Leu Gly Thr Pro Glu Lys Ile Ser Val Thr Tyr Pro Ser Leu
               100                 105                 110
   Ile Asp Asp Val Ser Val Gly Ala Lys Ile Leu Leu Asp Asp Gly Leu
           115                 120                 125
   Ile Ser Leu Glu Val Asn Ala Val Asp Lys Gln Ala Gly Glu Ile Val
       130                 135                 140
   Thr Thr Val Leu Asn Gly Gly Val Leu Lys Asn Lys Lys Gly Val Asn
   145                 150                 155                 160
   Val Pro Gly Val Lys Val Asn Leu Pro Gly Ile Thr Glu Lys Asp Arg
                   165                 170                 175
   Ala Asp Ile Leu Phe Gly Ile Arg Gln Gly Ile Asp Phe Ile Ala Ala
               180                 185                 190
   Ser Phe Val Arg Arg Ala Ser Asp Val Leu Glu Ile Arg Glu Leu Leu
           195                 200                 205
   Glu Ala His Asp Ala Leu His Ile Gln Ile Ile Ala Lys Ile Glu Asn
       210                 215                 220
   Glu Glu Gly Val Ala Asn Ile Asp Glu Ile Leu Glu Ala Ala Asp Gly
   225                 230                 235                 240
   Leu Met Val Ala Arg Gly Asp Leu Gly Val Glu Ile Pro Ala Glu Glu
                   245                 250                 255
   Val Pro Leu Ile Gln Lys Leu Leu Ile Lys Lys Cys Asn Met Leu Gly
               260                 265                 270
   Lys Pro Val Ile Thr Ala Thr Gln Met Leu Asp Ser Met Gln Arg Asn
           275                 280                 285
   Pro Arg Pro Thr Arg Ala Glu Ala Ser Asp Val Ala Asn Ala Ile Phe
       290                 295                 300
   Asp Gly Thr Asp Ala Val Met Leu Ser Gly Glu Thr Ala Ala Gly Gln
   305                 310                 315                 320
   Tyr Pro Val Glu Ala Val Lys Thr Met His Gln Ile Ala Leu Arg Thr
                   325                 330                 335
   Glu Gln Ala Leu Glu His Arg Asp Ile Leu Ser Gln Arg Thr Lys Glu
               340                 345                 350
   Ser Gln Thr Thr Ile Thr Asp Ala Ile Gly Gln Ser Val Ala His Thr
           355                 360                 365
   Ala Leu Asn Leu Asp Val Ala Ala Ile Val Thr Pro Thr Val Ser Gly
       370                 375                 380
   Lys Thr Pro Gln Met Val Ala Lys Tyr Arg Pro Lys Ala Pro Ile Ile
   385                 390                 395                 400
   Ala Val Thr Ser Asn Glu Ala Val Ser Arg Arg Leu Ala Leu Val Trp
                   405                 410                 415
   Gly Val Tyr Thr Lys Glu Ala Pro His Val Asn Thr Thr Asp Glu Met
               420                 425                 430
   Leu Asp Val Ala Val Asp Ala Ala Val Arg Ser Gly Leu Val Lys His
           435                 440                 445
   Gly Asp Leu Val Val Ile Thr Ala Gly Val Pro Val Gly Glu Thr Gly
       450                 455                 460
   Ser Thr Asn Leu Met Lys Val His Val Ile Ser Asp Leu Leu Ala Lys
   465                 470                 475                 480
   Gly Gln Gly Ile Gly Arg Lys Ser Ala Phe Gly Lys Ala Val Val Ala
                   485                 490                 495
   Lys Thr Ala Glu Glu Ala Arg Gln Lys Met Val Asp Gly Gly Ile Leu
               500                 505                 510
   Val Thr Val Ser Thr Asp Ala Asp Met Met Pro Ala Ile Glu Lys Ala
           515                 520                 525
   Ala Ala Ile Ile Thr Glu Glu Gly Gly Leu Thr Ser His Ala Ala Val
       530                 535                 540
   Val Gly Leu Ser Leu Gly Ile Pro Val Ile Val Gly Val Glu Asn Ala
   545                 550                 555                 560
   Thr Thr Leu Phe Lys Asp Gly Gln Glu Ile Thr Val Asp Gly Gly Phe
                   565                 570                 575
   Gly Ala Val Tyr Arg Gly His Ala Ser Val Leu
               580                 585
<210> 3
   <211> 244
   <212> PRT
   <213> Pyrococcus furiosus
<400> 3
   Gly Ser Ser His His His His His His Ser Ser Gly Leu Val Pro Arg
   1               5                   10                  15
   Gly Ser His Met Arg Ile Val Phe Asp Ile Gly Gly Ser Val Leu Val
               20                  25                  30
   Pro Glu Asn Pro Asp Ile Asp Phe Ile Lys Glu Ile Ala Tyr Gln Leu
           35                  40                  45
   Thr Lys Val Ser Glu Asp His Glu Val Ala Val Val Val Gly Gly Gly
       50                  55                  60
   Lys Leu Ala Arg Lys Tyr Ile Glu Val Ala Glu Lys Phe Asn Ser Ser
   65                  70                  75                  80
   Glu Thr Phe Lys Asp Phe Ile Gly Ile Gln Ile Thr Arg Ala Asn Ala
                   85                  90                  95
   Met Leu Leu Ile Ala Ala Leu Arg Glu Lys Ala Tyr Pro Val Val Val
               100                 105                 110
   Glu Asp Phe Trp Glu Ala Trp Lys Ala Val Gln Leu Lys Lys Ile Pro
           115                 120                 125
   Val Met Gly Gly Thr His Pro Gly His Thr Thr Asp Ala Val Ala Ala
       130                 135                 140
   Leu Leu Ala Glu Phe Leu Lys Ala Asp Leu Leu Val Val Ile Thr Asn
   145                 150                 155                 160
   Val Asp Gly Val Tyr Thr Ala Asp Pro Lys Lys Asp Pro Thr Ala Lys
                   165                 170                 175
   Lys Ile Lys Lys Met Lys Pro Glu Glu Leu Leu Glu Ile Val Gly Lys
               180                 185                 190
   Gly Ile Glu Lys Ala Gly Ser Ser Ser Val Ile Asp Pro Leu Ala Ala
           195                 200                 205
   Lys Ile Ile Ala Arg Ser Gly Ile Lys Thr Ile Val Ile Gly Lys Glu
       210                 215                 220
   Asp Ala Lys Asp Leu Phe Arg Val Ile Lys Gly Asp His Asn Gly Thr
   225                 230                 235                 240
   Thr Ile Glu Pro
<210> 4
   <211> 375
   <212> PRT
   <213> Thermotoga maritima
<400> 4
   Met Phe Arg Ile Leu Thr Ile Asn Pro Gly Ser Thr Ser Thr Lys Leu
   1               5                   10                  15
   Ser Ile Phe Glu Asp Glu Arg Met Val Lys Met Gln Asn Phe Ser His
               20                  25                  30
   Ser Pro Asp Glu Leu Gly Arg Phe Gln Lys Ile Leu Asp Gln Leu Glu
           35                  40                  45
   Phe Arg Glu Lys Ile Ala Arg Gln Phe Val Glu Glu Thr Gly Tyr Ser
       50                  55                  60
   Leu Ser Ser Phe Ser Ala Phe Val Ser Arg Gly Gly Leu Leu Asp Pro
   65                  70                  75                  80
   Ile Pro Gly Gly Val Tyr Leu Val Asp Gly Leu Met Ile Lys Thr Leu
   85 90 95
   Lys Ser Gly Lys Asn Gly Glu His Ala Ser Asn Leu Gly Ala Ile Ile
   100 105 110
   Ala His Arg Phe Ser Ser Glu Thr Gly Val Pro Ala Tyr Val Val Asp
   115 120 125
   Pro Val Val Val Asp Glu Met Glu Asp Val Ala Arg Val Ser Gly His
   130 135 140
   Pro Asn Tyr Gln Arg Lys Ser Ile Phe His Ala Leu Asn Gln Lys Thr
   145 150 155 160
   Val Ala Lys Glu Val Ala Arg Met Met Asn Lys Arg Tyr Glu Glu Met
   165 170 175
   Asn Leu Val Val Ala His Met Gly Gly Gly Ile Ser Ile Ala Ala His
   180 185 190
   Arg Lys Gly Arg Val Ile Asp Val Asn Asn Ala Leu Asp Gly Asp Gly
   195 200 205
   Pro Phe Thr Pro Glu Arg Ser Gly Thr Leu Pro Leu Thr Gln Leu Val
   210 215 220
   Asp Leu Cys Phe Ser Gly Lys Phe Thr Tyr Glu Glu Met Lys Lys Arg
   225 230 235 240
   Ile Val Gly Asn Gly Gly Leu Val Ala Tyr Leu Gly Thr Ser Asp Ala
   245 250 255
   Arg Glu Val Val Arg Arg Ile Lys Gln Gly Asp Glu Trp Ala Lys Arg
   260 265 270
   Val Tyr Arg Ala Met Ala Tyr Gln Ile Ala Lys Trp Ile Gly Lys Met
   275 280 285
   Ala Ala Val Leu Lys Gly Glu Val Asp Phe Ile Val Leu Thr Gly Gly
   290 295 300
   Leu Ala His Glu Lys Glu Phe Leu Val Pro Trp Ile Thr Lys Arg Val
   305 310 315 320
   Ser Phe Ile Ala Pro Val Leu Val Phe Pro Gly Ser Asn Glu Glu Lys
   325 330 335
   Ala Leu Ala Leu Ser Ala Leu Arg Val Leu Arg Gly Glu Glu Lys Pro
   340 345 350
   Lys Asn Tyr Ser Glu Glu Ser Arg Arg Trp Arg Glu Arg Tyr Asp Ser
   355 360 365
   Tyr Leu Asp Gly Ile Leu Arg
   370 375
<210> 5
   <211> 220
   <212> PRT
   <213> Thermotoga maritima
<400> 5
   Met Met Ala Tyr Leu Val Phe Leu Gly Pro Pro Gly Ala Gly Lys Gly
   1 5 10 15
   Thr Tyr Ala Lys Arg Leu Gln Glu Ile Thr Gly Ile Pro His Ile Ser
   20 25 30
   Thr Gly Asp Ile Phe Arg Asp Ile Val Lys Lys Glu Asn Asp Glu Leu
   35 40 45
   Gly Lys Lys Ile Lys Glu Ile Met Glu Arg Gly Glu Leu Val Pro Asp
   50 55 60
   Glu Leu Val Asn Glu Val Val Lys Arg Arg Leu Ser Glu Lys Asp Cys
   65 70 75 80
   Glu Arg Gly Phe Ile Leu Asp Gly Tyr Pro Arg Thr Val Ala Gln Ala
   85 90 95
   Glu Phe Leu Asp Gly Phe Leu Lys Thr Gln Asn Lys Glu Leu Thr Ala
   100 105 110
   Ala Val Leu Phe Glu Val Pro Glu Glu Val Val Val Gln Arg Leu Thr
   115 120 125
   Ala Arg Arg Ile Cys Pro Lys Cys Gly Arg Ile Tyr Asn Leu Ile Ser
   130 135 140
   Leu Pro Pro Lys Glu Asp Glu Leu Cys Asp Asp Cys Lys Val Lys Leu
   145 150 155 160
   Val Gln Arg Glu Asp Asp Lys Glu Glu Thr Val Arg His Arg Tyr Lys
   165 170 175
   Val Tyr Leu Glu Lys Thr Gln Pro Val Ile Asp Tyr Tyr Asp Lys Lys
   180 185 190
   Gly Ile Leu Lys Arg Val Asp Gly Thr Ile Gly Ile Asp Asn Val Ile
   195 200 205
   Ala Glu Val Leu Lys Ile Ile Gly Trp Ser Asp Lys
   210 215 220
<210> 6
   <211> 194
   <212> PRT
   <213> Sulfolobus acidocaldarius
<400> 6
   Met Lys Ile Gly Ile Val Thr Gly Ile Pro Gly Val Gly Lys Ser Thr
   1 5 10 15
   Val Leu Ala Lys Val Lys Glu Ile Leu Asp Asn Gln Gly Ile Asn Asn
   20 25 30
   Lys Ile Ile Asn Tyr Gly Asp Phe Met Leu Ala Thr Ala Leu Lys Leu
   35 40 45
   Gly Tyr Ala Lys Asp Arg Asp Glu Met Arg Lys Leu Ser Val Glu Lys
   50 55 60
   Gln Lys Lys Leu Gln Ile Asp Ala Ala Lys Gly Ile Ala Glu Glu Ala
   65 70 75 80
   Arg Ala Gly Gly Glu Gly Tyr Leu Phe Ile Asp Thr His Ala Val Ile
   85 90 95
   Arg Thr Pro Ser Gly Tyr Leu Pro Gly Leu Pro Ser Tyr Val Ile Thr
   100 105 110
   Glu Ile Asn Pro Ser Val Ile Phe Leu Leu Glu Ala Asp Pro Lys Ile
   115 120 125
   Ile Leu Ser Arg Gln Lys Arg Asp Thr Thr Arg Asn Arg Asn Asp Tyr
   130 135 140
   Ser Asp Glu Ser Val Ile Leu Glu Thr Ile Asn Phe Ala Arg Tyr Ala
   145 150 155 160
   Ala Thr Ala Ser Ala Val Leu Ala Gly Ser Thr Val Lys Val Ile Val
   165 170 175
   Asn Val Glu Gly Asp Pro Ser Ile Ala Ala Asn Glu Ile Ile Arg Ser
   180 185 190
   Met Lys
<210> 7
   <211> 981
   <212> DNA
   <213> Artificial Sequence
<220>
   <223> Adenylate kinase from Sulfolobus acidocaldarius with unstructured linker and VNAR sequence
<400> 7
   atgaagatcg gcattgtgac cggcattccg ggcgttggca aaagcaccgt tctggcaaag      60
   gtgaaggaga tcctggacaa ccagggcatt aataacaaaa ttattaatta tggtgatttt     120
   atgctggcga ccgcgctgaa gctgggctac gcaaaagatc gtgacgaaat gcgcaaactg     180
   agcgtggaaa aacagaagaa gctgcagatt gatgcggcga agggcattgc ggaagaggca     240
   cgcgcgggcg gcgaaggcta cctgtttatc gatacccatg cggtgatccg caccccgagc     300
   ggttatctgc cgggcctgcc gtcttacgtg attacggaaa tcaacccgag cgttattttt     360
   ctgctggagg cagatccgaa gattattctg agccgccaga agcgcgatac cacccgcaac     420
   cgcaacgatt atagcgacga aagcgttatc ctggagacca tcaactttgc gcgctatgcg     480
   gcaaccgcga gcgcggttct ggcaggctct accgttaaag tgatcgtgaa cgtggagggt     540
   gatccaagca tcgcggcgaa cgaaatcatt cgcagcatga aacagtcgac cggtggtggt     600
   ggttctggtg gtggtggttc tggtggtggt ggttctggat ccgcacgtgt tgatcagaca     660
   ccgcgtagcg ttaccaaaga aaccggtgaa agcctgacca ttaattgtgt tctgcgtgat     720
   gcaagctatg cactgggtag cacctgttgg tatcgtaaaa aaagcggcag caccaatgaa     780
   gaaagcatta gcaaaggtgg tcgttatgtt gaaaccgtga atagcggtag caaaagcttt     840
   agcctgcgta ttaatgatct gaccgttgaa gatggtggga cctaccgttg tggtctgggt     900
   gttgccggtg gttattgtga ttatgcactg tgcagcagcc gttatgcaga atgtggtgat     960
   ggtactgcag ttaccgtgaa t                                               981
<210> 8
   <211> 115
   <212> PRT
   <213> Artificial Sequence
<220>
   <223> VNAR sequence for hen egg lysozyme specific single domain antibody from shark
<400> 8
   Gly Ser Ala Arg Val Asp Gln Thr Pro Arg Ser Val Thr Lys Glu Thr
   1 5 10 15
   Gly Glu Ser Leu Thr Ile Asn Cys Val Leu Arg Asp Ala Ser Tyr Ala
   20 25 30
   Leu Gly Ser Thr Cys Trp Tyr Arg Lys Lys Ser Gly Ser Thr Asn Glu
   35 40 45
   Glu Ser Ile Ser Lys Gly Gly Arg Tyr Val Glu Thr Val Asn Ser Gly
   50 55 60
   Ser Lys Ser Phe Ser Leu Arg Ile Asn Asp Leu Thr Val Glu Asp Gly
   65 70 75 80
   Gly Thr Tyr Arg Cys Gly Leu Gly Val Ala Gly Gly Tyr Cys Asp Tyr
   85 90 95
   Ala Leu Cys Ser Ser Arg Tyr Ala Glu Cys Gly Asp Gly Thr Ala Val
   100 105 110
   Thr Val Asn
   115
<210> 9
   <211> 287
   <212> PRT
   <213> Artificial Sequence
<220>
   <223> Fusion protein sequence for adenylate kinase from Sulfolobus acidocaldarius with unstructured linker and VNAR sequence
<400> 9
   Met Leu Ala Thr Ala Leu Lys Leu Gly Tyr Ala Lys Asp Arg Asp Glu
   1 5 10 15
   Met Arg Lys Leu Ser Val Glu Lys Gln Lys Lys Leu Gln Ile Asp Ala
   20 25 30
   Ala Lys Gly Ile Ala Glu Glu Ala Arg Ala Gly Gly Glu Gly Tyr Leu
   35 40 45
   Phe Ile Asp Thr His Ala Val Ile Arg Thr Pro Ser Gly Tyr Leu Pro
   50 55 60
   Gly Leu Pro Ser Tyr Val Ile Thr Glu Ile Asn Pro Ser Val Ile Phe
   65 70 75 80
   Leu Leu Glu Ala Asp Pro Lys Ile Ile Leu Ser Arg Gln Lys Arg Asp
   85 90 95
   Thr Thr Arg Asn Arg Asn Asp Tyr Ser Asp Glu Ser Val Ile Leu Glu
   100 105 110
   Thr Ile Asn Phe Ala Arg Tyr Ala Ala Thr Ala Ser Ala Val Leu Ala
   115 120 125
   Gly Ser Thr Val Lys Val Ile Val Asn Val Glu Gly Asp Pro Ser Ile
   130 135 140
   Ala Ala Asn Glu Ile Ile Arg Ser Met Lys Gln Ser Thr Gly Gly Gly
   145 150 155 160
   Gly Ser Gly Gly Gly Gly Ser Gly Gly Gly Gly Ser Gly Ser Ala Arg
   165 170 175
   Val Asp Gln Thr Pro Arg Ser Val Thr Lys Glu Thr Gly Glu Ser Leu
   180 185 190
   Thr Ile Asn Cys Val Leu Arg Asp Ala Ser Tyr Ala Leu Gly Ser Thr
   195 200 205
   Cys Trp Tyr Arg Lys Lys Ser Gly Ser Thr Asn Glu Glu Ser Ile Ser
   210 215 220
   Lys Gly Gly Arg Tyr Val Glu Thr Val Asn Ser Gly Ser Lys Ser Phe
   225 230 235 240
   Ser Leu Arg Ile Asn Asp Leu Thr Val Glu Asp Gly Gly Thr Tyr Arg
   245 250 255
   Cys Gly Leu Gly Val Ala Gly Gly Tyr Cys Asp Tyr Ala Leu Cys Ser
   260 265 270
   Ser Arg Tyr Ala Glu Cys Gly Asp Gly Thr Ala Val Thr Val Asn
   275 280 285
<210> 10
   <211> 297
   <212> PRT
   <213> Artificial Sequence
<220>
   <223> Fusion protein sequence for adenylate kinase from Sulfolobus acidocaldarius with helical linker and VNAR sequence
<400> 10
   Met Leu Ala Thr Ala Leu Lys Leu Gly Tyr Ala Lys Asp Arg Asp Glu
   1               5                   10                  15
   Met Arg Lys Leu Ser Val Glu Lys Gln Lys Lys Leu Gln Ile Asp Ala
               20                  25                  30
   Ala Lys Gly Ile Ala Glu Glu Ala Arg Ala Gly Gly Glu Gly Tyr Leu
           35                  40                  45
   Phe Ile Asp Thr His Ala Val Ile Arg Thr Pro Ser Gly Tyr Leu Pro
       50                  55                  60
   Gly Leu Pro Ser Tyr Val Ile Thr Glu Ile Asn Pro Ser Val Ile Phe
   65                  70                  75                  80
   Leu Leu Glu Ala Asp Pro Lys Ile Ile Leu Ser Arg Gln Lys Arg Asp
                   85                  90                  95
   Thr Thr Arg Asn Arg Asn Asp Tyr Ser Asp Glu Ser Val Ile Leu Glu
               100                 105                 110
   Thr Ile Asn Phe Ala Arg Tyr Ala Ala Thr Ala Ser Ala Val Leu Ala
           115                 120                 125
   Gly Ser Thr Val Lys Val Ile Val Asn Val Glu Gly Asp Pro Ser Ile
       130                 135                 140
   Ala Ala Asn Glu Ile Ile Arg Ser Met Lys Gln Ser Thr Leu Ala Glu
   145                 150                 155                 160
   Ala Ala Ala Lys Glu Ala Ala Ala Lys Glu Ala Ala Ala Lys Glu Ala
                   165                 170                 175
   Ala Ala Lys Ala Ala Ala Gly Ser Ala Arg Val Asp Gln Thr Pro Arg
               180                 185                 190
   Ser Val Thr Lys Glu Thr Gly Glu Ser Leu Thr Ile Asn Cys Val Leu
           195                 200                 205
   Arg Asp Ala Ser Tyr Ala Leu Gly Ser Thr Cys Trp Tyr Arg Lys Lys
       210                 215                 220
   Ser Gly Ser Thr Asn Glu Glu Ser Ile Ser Lys Gly Gly Arg Tyr Val
   225                 230                 235                 240
   Glu Thr Val Asn Ser Gly Ser Lys Ser Phe Ser Leu Arg Ile Asn Asp
   245 250 255
   Leu Thr Val Glu Asp Gly Gly Thr Tyr Arg Cys Gly Leu Gly Val Ala
   260 265 270
   Gly Gly Tyr Cys Asp Tyr Ala Leu Cys Ser Ser Arg Tyr Ala Glu Cys
   275 280 285
   Gly Asp Gly Thr Ala Val Thr Val Asn
   290 295
<210> 11
   <211> 299
   <212> PRT
   <213> Artificial Sequence
<220>
   <223> Fusion protein sequence for VNAR sequence with helical linker and adenylate kinase from Sulfolobus acidocaldarius
<400> 11
   Met Gly Ser Ala Arg Val Asp Gln Thr Pro Arg Ser Val Thr Lys Glu
   1 5 10 15
   Thr Gly Glu Ser Leu Thr Ile Asn Cys Val Leu Arg Asp Ala Ser Tyr
   20 25 30
   Ala Leu Gly Ser Thr Cys Trp Tyr Arg Lys Lys Ser Gly Ser Thr Asn
   35 40 45
   Glu Glu Ser Ile Ser Lys Gly Gly Arg Tyr Val Glu Thr Val Asn Ser
   50 55 60
   Gly Ser Lys Ser Phe Ser Leu Arg Ile Asn Asp Leu Thr Val Glu Asp
   65 70 75 80
   Gly Gly Thr Tyr Arg Cys Gly Leu Gly Val Ala Gly Gly Tyr Cys Asp
   85 90 95
   Tyr Ala Leu Cys Ser Ser Arg Tyr Ala Glu Cys Gly Asp Gly Thr Ala
   100 105 110
   Val Thr Val Asn Ser Thr Leu Ala Glu Ala Ala Ala Lys Glu Ala Ala
   115 120 125
   Ala Lys Glu Ala Ala Ala Lys Glu Ala Ala Ala Lys Ala Ala Ala Gly
   130 135 140
   Ser Met Leu Ala Thr Ala Leu Lys Leu Gly Tyr Ala Lys Asp Arg Asp
   145 150 155 160
   Glu Met Arg Lys Leu Ser Val Glu Lys Gln Lys Lys Leu Gln Ile Asp
   165 170 175
   Ala Ala Lys Gly Ile Ala Glu Glu Ala Arg Ala Gly Gly Glu Gly Tyr
   180 185 190
   Leu Phe Ile Asp Thr His Ala Val Ile Arg Thr Pro Ser Gly Tyr Leu
   195 200 205
   Pro Gly Leu Pro Ser Tyr Val Ile Thr Glu Ile Asn Pro Ser Val Ile
   210 215 220
   Phe Leu Leu Glu Ala Asp Pro Lys Ile Ile Leu Ser Arg Gln Lys Arg
   225 230 235 240
   Asp Thr Thr Arg Asn Arg Asn Asp Tyr Ser Asp Glu Ser Val Ile Leu
   245 250 255
   Glu Thr Ile Asn Phe Ala Arg Tyr Ala Ala Thr Ala Ser Ala Val Leu
   260 265 270
   Ala Gly Ser Thr Val Lys Val Ile Val Asn Val Glu Gly Asp Pro Ser
   275 280 285
   Ile Ala Ala Asn Glu Ile Ile Arg Ser Met Lys
   290 295
<210> 12
   <211> 289
   <212> PRT
   <213> Artificial Sequence
<220>
   <223> Fusion protein sequence for VNAR sequence with unstructured linker and adenylate kinase from Sulfolobus acidocaldarius
<400> 12
   Met Gly Ser Ala Arg Val Asp Gln Thr Pro Arg Ser Val Thr Lys Glu
   1 5 10 15
   Thr Gly Glu Ser Leu Thr Ile Asn Cys Val Leu Arg Asp Ala Ser Tyr
   20 25 30
   Ala Leu Gly Ser Thr Cys Trp Tyr Arg Lys Lys Ser Gly Ser Thr Asn
   35 40 45
   Glu Glu Ser Ile Ser Lys Gly Gly Arg Tyr Val Glu Thr Val Asn Ser
       50                  55                  60
   Gly Ser Lys Ser Phe Ser Leu Arg Ile Asn Asp Leu Thr Val Glu Asp
   65                  70                  75                  80
   Gly Gly Thr Tyr Arg Cys Gly Leu Gly Val Ala Gly Gly Tyr Cys Asp
                   85                  90                  95
   Tyr Ala Leu Cys Ser Ser Arg Tyr Ala Glu Cys Gly Asp Gly Thr Ala
               100                 105                 110
   Val Thr Val Asn Ser Thr Gly Gly Gly Gly Ser Gly Gly Gly Gly Ser
           115                 120                 125
   Gly Gly Gly Gly Ser Gly Ser Met Leu Ala Thr Ala Leu Lys Leu Gly
       130                 135                 140
   Tyr Ala Lys Asp Arg Asp Glu Met Arg Lys Leu Ser Val Glu Lys Gln
   145                 150                 155                 160
   Lys Lys Leu Gln Ile Asp Ala Ala Lys Gly Ile Ala Glu Glu Ala Arg
                   165                 170                 175
   Ala Gly Gly Glu Gly Tyr Leu Phe Ile Asp Thr His Ala Val Ile Arg
               180                 185                 190
   Thr Pro Ser Gly Tyr Leu Pro Gly Leu Pro Ser Tyr Val Ile Thr Glu
           195                 200                 205
   Ile Asn Pro Ser Val Ile Phe Leu Leu Glu Ala Asp Pro Lys Ile Ile
       210                 215                 220
   Leu Ser Arg Gln Lys Arg Asp Thr Thr Arg Asn Arg Asn Asp Tyr Ser
   225                 230                 235                 240
   Asp Glu Ser Val Ile Leu Glu Thr Ile Asn Phe Ala Arg Tyr Ala Ala
                   245                 250                 255
   Thr Ala Ser Ala Val Leu Ala Gly Ser Thr Val Lys Val Ile Val Asn
               260                 265                 270
   Val Glu Gly Asp Pro Ser Ile Ala Ala Asn Glu Ile Ile Arg Ser Met
           275                 280                 285
   Lys
<210> 13
   <211> 128
   <212> PRT
   <213> Artificial Sequence
<220>
   <223> VHH sequence for hen egg lysozyme specific single domain antibody from camelid
<400> 13
   Glu Val Gln Leu Gln Ala Ser Gly Gly Gly Ser Val Gln Ala Gly Gln
   1 5 10 15
   Ser Leu Arg Leu Ser Cys Ala Thr Ser Gly Ala Thr Ser Ser Ser Asn
   20 25 30
   Cys Met Gly Trp Phe Arg Gln Ala Pro Gly Lys Glu Arg Glu Gly Val
   35 40 45
   Ala Val Ile Asp Thr Gly Arg Gly Asn Thr Ala Tyr Ala Asp Ser Val
   50 55 60
   Gln Gly Arg Leu Thr Ile Ser Leu Asp Asn Ala Lys Asn Thr Leu Tyr
   65 70 75 80
   Leu Gln Met Asn Ser Leu Lys Pro Glu Asp Thr Ala Met Tyr Tyr Cys
   85 90 95
   Ala Ala Asp Thr Ser Thr Trp Tyr Arg Gly Tyr Cys Gly Thr Asn Pro
   100 105 110
   Asn Tyr Phe Ser Tyr Trp Gly Gln Gly Thr Gln Val Thr Val Ser Ser
   115 120 125
<210> 14
   <211> 302
   <212> PRT
   <213> Artificial Sequence
<220>
   <223> Fusion protein sequence for adenylate kinase from Sulfolobus acidocaldarius with unstructured linker and VHH sequence
<400> 14
   Met Leu Ala Thr Ala Leu Lys Leu Gly Tyr Ala Lys Asp Arg Asp Glu
   1 5 10 15
   Met Arg Lys Leu Ser Val Glu Lys Gln Lys Lys Leu Gln Ile Asp Ala
   20 25 30
   Ala Lys Gly Ile Ala Glu Glu Ala Arg Ala Gly Gly Glu Gly Tyr Leu
   35 40 45
   Phe Ile Asp Thr His Ala Val Ile Arg Thr Pro Ser Gly Tyr Leu Pro
   50 55 60
   Gly Leu Pro Ser Tyr Val Ile Thr Glu Ile Asn Pro Ser Val Ile Phe
   65 70 75 80
   Leu Leu Glu Ala Asp Pro Lys Ile Ile Leu Ser Arg Gln Lys Arg Asp
   85 90 95
   Thr Thr Arg Asn Arg Asn Asp Tyr Ser Asp Glu Ser Val Ile Leu Glu
   100 105 110
   Thr Ile Asn Phe Ala Arg Tyr Ala Ala Thr Ala Ser Ala Val Leu Ala
   115 120 125
   Gly Ser Thr Val Lys Val Ile Val Asn Val Glu Gly Asp Pro Ser Ile
   130 135 140
   Ala Ala Asn Glu Ile Ile Arg Ser Met Lys Gln Ser Thr Gly Gly Gly
   145 150 155 160
   Gly Ser Gly Gly Gly Gly Ser Gly Gly Gly Gly Ser Gly Ser Glu Val
   165 170 175
   Gln Leu Gln Ala Ser Gly Gly Gly Ser Val Gln Ala Gly Gln Ser Leu
   180 185 190
   Arg Leu Ser Cys Ala Thr Ser Gly Ala Thr Ser Ser Ser Asn Cys Met
   195 200 205
   Gly Trp Phe Arg Gln Ala Pro Gly Lys Glu Arg Glu Gly Val Ala Val
   210 215 220
   Ile Asp Thr Gly Arg Gly Asn Thr Ala Tyr Ala Asp Ser Val Gln Gly
   225 230 235 240
   Arg Leu Thr Ile Ser Leu Asp Asn Ala Lys Asn Thr Leu Tyr Leu Gln
   245 250 255
   Met Asn Ser Leu Lys Pro Glu Asp Thr Ala Met Tyr Tyr Cys Ala Ala
   260 265 270
   Asp Thr Ser Thr Trp Tyr Arg Gly Tyr Cys Gly Thr Asn Pro Asn Tyr
   275 280 285
   Phe Ser Tyr Trp Gly Gln Gly Thr Gln Val Thr Val Ser Ser
   290 295 300
<210> 15
   <211> 312
   <212> PRT
   <213> Artificial Sequence
<220>
   <223> Fusion protein sequence for adenylate kinase from Sulfolobus acidocaldarius with helical linker and VHH sequence
<400> 15
   Met Leu Ala Thr Ala Leu Lys Leu Gly Tyr Ala Lys Asp Arg Asp Glu
   1 5 10 15
   Met Arg Lys Leu Ser Val Glu Lys Gln Lys Lys Leu Gln Ile Asp Ala
   20 25 30
   Ala Lys Gly Ile Ala Glu Glu Ala Arg Ala Gly Gly Glu Gly Tyr Leu
   35 40 45
   Phe Ile Asp Thr His Ala Val Ile Arg Thr Pro Ser Gly Tyr Leu Pro
   50 55 60
   Gly Leu Pro Ser Tyr Val Ile Thr Glu Ile Asn Pro Ser Val Ile Phe
   65 70 75 80
   Leu Leu Glu Ala Asp Pro Lys Ile Ile Leu Ser Arg Gln Lys Arg Asp
   85 90 95
   Thr Thr Arg Asn Arg Asn Asp Tyr Ser Asp Glu Ser Val Ile Leu Glu
   100 105 110
   Thr Ile Asn Phe Ala Arg Tyr Ala Ala Thr Ala Ser Ala Val Leu Ala
   115 120 125
   Gly Ser Thr Val Lys Val Ile Val Asn Val Glu Gly Asp Pro Ser Ile
   130 135 140
   Ala Ala Asn Glu Ile Ile Arg Ser Met Lys Gln Ser Thr Leu Ala Glu
   145 150 155 160
   Ala Ala Ala Lys Glu Ala Ala Ala Lys Glu Ala Ala Ala Lys Glu Ala
   165 170 175
   Ala Ala Lys Ala Ala Ala Gly Ser Glu Val Gln Leu Gln Ala Ser Gly
   180 185 190
   Gly Gly Ser Val Gln Ala Gly Gln Ser Leu Arg Leu Ser Cys Ala Thr
   195 200 205
   Ser Gly Ala Thr Ser Ser Ser Asn Cys Met Gly Trp Phe Arg Gln Ala
   210 215 220
   Pro Gly Lys Glu Arg Glu Gly Val Ala Val Ile Asp Thr Gly Arg Gly
   225 230 235 240
   Asn Thr Ala Tyr Ala Asp Ser Val Gln Gly Arg Leu Thr Ile Ser Leu
   245 250 255
   Asp Asn Ala Lys Asn Thr Leu Tyr Leu Gln Met Asn Ser Leu Lys Pro
   260 265 270
   Glu Asp Thr Ala Met Tyr Tyr Cys Ala Ala Asp Thr Ser Thr Trp Tyr
   275 280 285
   Arg Gly Tyr Cys Gly Thr Asn Pro Asn Tyr Phe Ser Tyr Trp Gly Gln
   290 295 300
   Gly Thr Gln Val Thr Val Ser Ser
   305 310
<210> 16
   <211> 312
   <212> PRT
   <213> Artificial Sequence
<220>
   <223> Fusion protein sequence for VHH sequence with helical linker and adenylate kinase from Sulfolobus acidocaldarius
<400> 16
   Met Glu Val Gln Leu Gln Ala Ser Gly Gly Gly Ser Val Gln Ala Gly
   1 5 10 15
   Gln Ser Leu Arg Leu Ser Cys Ala Thr Ser Gly Ala Thr Ser Ser Ser
   20 25 30
   Asn Cys Met Gly Trp Phe Arg Gln Ala Pro Gly Lys Glu Arg Glu Gly
   35 40 45
   Val Ala Val Ile Asp Thr Gly Arg Gly Asn Thr Ala Tyr Ala Asp Ser
   50 55 60
   Val Gln Gly Arg Leu Thr Ile Ser Leu Asp Asn Ala Lys Asn Thr Leu
   65 70 75 80
   Tyr Leu Gln Met Asn Ser Leu Lys Pro Glu Asp Thr Ala Met Tyr Tyr
                   85                  90                  95
   Cys Ala Ala Asp Thr Ser Thr Trp Tyr Arg Gly Tyr Cys Gly Thr Asn
               100                 105                 110
   Pro Asn Tyr Phe Ser Tyr Trp Gly Gln Gly Thr Gln Val Thr Val Ser
           115                 120                 125
   Ser Ser Thr Leu Ala Glu Ala Ala Ala Lys Glu Ala Ala Ala Lys Glu
       130                 135                 140
   Ala Ala Ala Lys Glu Ala Ala Ala Lys Ala Ala Ala Gly Ser Met Leu
   145                 150                 155                 160
   Ala Thr Ala Leu Lys Leu Gly Tyr Ala Lys Asp Arg Asp Glu Met Arg
                   165                 170                 175
   Lys Leu Ser Val Glu Lys Gln Lys Lys Leu Gln Ile Asp Ala Ala Lys
               180                 185                 190
   Gly Ile Ala Glu Glu Ala Arg Ala Gly Gly Glu Gly Tyr Leu Phe Ile
           195                 200                 205
   Asp Thr His Ala Val Ile Arg Thr Pro Ser Gly Tyr Leu Pro Gly Leu
       210                 215                 220
   Pro Ser Tyr Val Ile Thr Glu Ile Asn Pro Ser Val Ile Phe Leu Leu
   225                 230                 235                 240
   Glu Ala Asp Pro Lys Ile Ile Leu Ser Arg Gln Lys Arg Asp Thr Thr
                   245                 250                 255
   Arg Asn Arg Asn Asp Tyr Ser Asp Glu Ser Val Ile Leu Glu Thr Ile
               260                 265                 270
   Asn Phe Ala Arg Tyr Ala Ala Thr Ala Ser Ala Val Leu Ala Gly Ser
           275                 280                 285
   Thr Val Lys Val Ile Val Asn Val Glu Gly Asp Pro Ser Ile Ala Ala
       290                 295                 300
   Asn Glu Ile Ile Arg Ser Met Lys
   305                 310
<210> 17
   <211> 301
   <212> PRT
   <213> Artificial Sequence
<220>
   <223> Fusion protein sequence for VHH sequence with unstructured linker and adenylate kinase from Sulfolobus acidocaldarius
<400> 17
   Glu Val Gln Leu Gln Ala Ser Gly Gly Gly Ser Val Gln Ala Gly Gln
   1               5                   10                  15
   Ser Leu Arg Leu Ser Cys Ala Thr Ser Gly Ala Thr Ser Ser Ser Asn
               20                  25                  30
   Cys Met Gly Trp Phe Arg Gln Ala Pro Gly Lys Glu Arg Glu Gly Val
           35                  40                  45
   Ala Val Ile Asp Thr Gly Arg Gly Asn Thr Ala Tyr Ala Asp Ser Val
       50                  55                  60
   Gln Gly Arg Leu Thr Ile Ser Leu Asp Asn Ala Lys Asn Thr Leu Tyr
   65                  70                  75                  80
   Leu Gln Met Asn Ser Leu Lys Pro Glu Asp Thr Ala Met Tyr Tyr Cys
                   85                  90                  95
   Ala Ala Asp Thr Ser Thr Trp Tyr Arg Gly Tyr Cys Gly Thr Asn Pro
               100                 105                 110
   Asn Tyr Phe Ser Tyr Trp Gly Gln Gly Thr Gln Val Thr Val Ser Ser
           115                 120                 125
   Ser Thr Gly Gly Gly Gly Ser Gly Gly Gly Gly Ser Gly Gly Gly Gly
       130                 135                 140
   Ser Gly Ser Met Leu Ala Thr Ala Leu Lys Leu Gly Tyr Ala Lys Asp
   145                 150                 155                 160
   Arg Asp Glu Met Arg Lys Leu Ser Val Glu Lys Gln Lys Lys Leu Gln
                   165                 170                 175
   Ile Asp Ala Ala Lys Gly Ile Ala Glu Glu Ala Arg Ala Gly Gly Glu
               180                 185                 190
   Gly Tyr Leu Phe Ile Asp Thr His Ala Val Ile Arg Thr Pro Ser Gly
           195                 200                 205
   Tyr Leu Pro Gly Leu Pro Ser Tyr Val Ile Thr Glu Ile Asn Pro Ser
       210                 215                 220
   Val Ile Phe Leu Leu Glu Ala Asp Pro Lys Ile Ile Leu Ser Arg Gln
   225 230 235 240
   Lys Arg Asp Thr Thr Arg Asn Arg Asn Asp Tyr Ser Asp Glu Ser Val
   245 250 255
   Ile Leu Glu Thr Ile Asn Phe Ala Arg Tyr Ala Ala Thr Ala Ser Ala
   260 265 270
   Val Leu Ala Gly Ser Thr Val Lys Val Ile Val Asn Val Glu Gly Asp
   275 280 285
   Pro Ser Ile Ala Ala Asn Glu Ile Ile Arg Ser Met Lys
   290 295 300
<210> 18
   <211> 25
   <212> PRT
   <213> Artificial Sequence
<220>
   <223> Helical linker
<400> 18
   Leu Ala Glu Ala Ala Ala Lys Glu Ala Ala Ala Lys Glu Ala Ala Ala
   1 5 10 15
   Lys Glu Ala Ala Ala Lys Ala Ala Ala
   20 25
<210> 19
   <211> 403
   <212> PRT
   <213> Thermotoga maritima
<400> 19
   Met Arg Val Leu Val Ile Asn Ser Gly Ser Ser Ser Ile Lys Tyr Gln
   1 5 10 15
   Leu Ile Glu Met Glu Gly Glu Lys Val Leu Cys Lys Gly Ile Ala Glu
   20 25 30
   Arg Ile Gly Ile Glu Gly Ser Arg Leu Val His Arg Val Gly Asp Glu
   35 40 45
   Lys His Val Ile Glu Arg Glu Leu Pro Asp His Glu Glu Ala Leu Lys
   50 55 60
   Leu Ile Leu Asn Thr Leu Val Asp Glu Lys Leu Gly Val Ile Lys Asp
   65 70 75 80
   Leu Lys Glu Ile Asp Ala Val Gly His Arg Val Val His Gly Gly Glu
   85 90 95
   Arg Phe Lys Glu Ser Val Leu Val Asp Glu Glu Val Leu Lys Ala Ile
   100 105 110
   Glu Glu Val Ser Pro Leu Ala Pro Leu His Asn Pro Ala Asn Leu Met
   115 120 125
   Gly Ile Lys Ala Ala Met Lys Leu Leu Pro Gly Val Pro Asn Val Ala
   130 135 140
   Val Phe Asp Thr Ala Phe His Gln Thr Ile Pro Gln Lys Ala Tyr Leu
   145 150 155 160
   Tyr Ala Ile Pro Tyr Glu Tyr Tyr Glu Lys Tyr Lys Ile Arg Arg Tyr
   165 170 175
   Gly Phe His Gly Thr Ser His Arg Tyr Val Ser Lys Arg Ala Ala Glu
   180 185 190
   Ile Leu Gly Lys Lys Leu Glu Glu Leu Lys Ile Ile Thr Cys His Ile
   195 200 205
   Gly Asn Gly Ala Ser Val Ala Ala Val Lys Tyr Gly Lys Cys Val Asp
   210 215 220
   Thr Ser Met Gly Phe Thr Pro Leu Glu Gly Leu Val Met Gly Thr Arg
   225 230 235 240
   Ser Gly Asp Leu Asp Pro Ala Ile Pro Phe Phe Ile Met Glu Lys Glu
   245 250 255
   Gly Ile Ser Pro Gln Glu Met Tyr Asp Ile Leu Asn Lys Lys Ser Gly
   260 265 270
   Val Tyr Gly Leu Ser Lys Gly Phe Ser Ser Asp Met Arg Asp Ile Glu
   275 280 285
   Glu Ala Ala Leu Lys Gly Asp Glu Trp Cys Lys Leu Val Leu Glu Ile
   290 295 300
   Tyr Asp Tyr Arg Ile Ala Lys Tyr Ile Gly Ala Tyr Ala Ala Ala Met
   305 310 315 320
   Asn Gly Val Asp Ala Ile Val Phe Thr Ala Gly Val Gly Glu Asn Ser
   325 330 335
   Pro Ile Thr Arg Glu Asp Val Cys Ser Tyr Leu Glu Phe Leu Gly Val
   340 345 350
   Lys Leu Asp Lys Gln Lys Asn Glu Glu Thr Ile Arg Gly Lys Glu Gly
   355 360 365
   Ile Ile Ser Thr Pro Asp Ser Arg Val Lys Val Leu Val Val Pro Thr
   370 375 380
   Asn Glu Glu Leu Met Ile Ala Arg Asp Thr Lys Glu Ile Val Glu Lys
   385 390 395 400
   Ile Gly Arg
<210> 20
   <211> 231
   <212> PRT
   <213> Thermotoga maritima
<400> 20
   Met Arg Val Leu Val Lys Leu Ser Gly Glu Ala Leu Ser Gly Glu Gly
   1 5 10 15
   Gly Arg Gly Phe Asp Pro Glu Arg Val Asn Tyr Ile Val Asn Glu Ile
   20 25 30
   Lys Ser Ala Ile Glu Glu Gly Phe Lys Ile Gly Ile Val Val Gly Ala
   35 40 45
   Gly Asn Leu Phe Arg Gly Val Glu Leu Lys Asn Leu Thr Met Thr Arg
   50 55 60
   Ala Asp Gln Ile Gly Leu Leu Gly Thr Val Met Asn Ser Val Tyr Leu
   65 70 75 80
   Lys Asp Ile Phe Glu Arg Ser Gly Leu Lys Ala Arg Ile Tyr Ser Gln
   85 90 95
   Ile Val Asn Leu Pro Asp Val Glu Arg Val Asn Tyr Asp Ser Ile Glu
   100 105 110
   Ser Ala Leu Arg Glu Asn Ser Ile Leu Ile Phe Ala Gly Gly Thr Ser
   115 120 125
   Asn Pro Phe Phe Thr Thr Asp Thr Ala Ala Val Leu Arg Ala Gln Glu
   130 135 140
   Met Arg Ala Lys Leu Val Val Lys Ala Thr Lys Val Asp Gly Val Tyr
   145 150 155 160
   Asp Lys Asp Pro Lys Lys Phe Pro Asp Ala Lys Lys Ile Pro His Leu
   165 170 175
   Thr Phe Ser Glu Ala Met Lys Met Gly Leu Lys Val Met Asp Ala Glu
   180 185 190
   Ala Phe Ala Leu Cys Lys Lys Leu Gly Ile Thr Val Lys Val Ile Asn
   195 200 205
   Phe Phe Glu Pro Gly Thr Leu Leu Lys Ala Leu Lys Gly Glu Asp Val
   210 215 220
   Gly Ser Thr Val Val Pro Asp
   225 230
<210> 21
   <211> 227
   <212> PRT
   <213> Sulfolobus acidocaldarius
<400> 21
   Met Lys Leu Thr Leu Lys Val Ser Gly Lys Phe Phe Asp Glu Glu Asn
   1 5 10 15
   Ser Glu Asn Leu Ser Leu Leu Arg Asp Val Ile Ile Asp Leu Val Asn
   20 25 30
   Asn Gly His Arg Val Ala Val Val Thr Gly Gly Gly Gly Thr Ala Arg
   35 40 45
   Arg Tyr Ile Ser Met Gly Arg Lys Leu Asn Leu Asn Glu Ser His Leu
   50 55 60
   Asp Ile Leu Gly Ile Leu Val Ser Arg Leu Asn Ala Gln Leu Leu Leu
   65 70 75 80
   Phe Ser Leu Asp Asn Ile Ala Tyr Pro Lys Val Pro Glu Ser Ile Glu
   85 90 95
   Asp Phe Asn Glu Arg Trp Ala Ser Gly Lys Val Val Ile Thr Gly Gly
   100 105 110
   Phe Gln Pro Gly Gln Ser Thr Ala Gly Val Ala Ala Leu Val Ser Glu
           115                 120                 125
   Ile Ile Asn Ala Asp Tyr Leu Val Leu Ala Thr Asn Val Asn Gly Val
       130                 135                 140
   Tyr Thr Lys Asp Pro Gln Lys Phe Val Asp Ala Lys Leu Leu Pro Lys
   145                 150                 155                 160
   Leu Thr Val Ser Glu Leu Lys Thr Ile Leu Glu Gly Ser Gln Ser Val
                   165                 170                 175
   Asn Ala Gly Lys Tyr Glu Leu Leu Asp Pro Leu Ala Ile Lys Ile Val
               180                 185                 190
   Glu Arg Ser Lys Ile Lys Val Leu Val Ile Asn Phe Lys Asp Leu Asn
           195                 200                 205
   Lys Leu Pro Asn Ile Leu Lys Gly Asn Glu Ile Leu Gly Ser Val Val
       210                 215                 220
   Val Pro Glu
   225

## Description

The invention relates to the expression and production of highly stable assay reagents comprising single-domain antibodies or antibody fragments, and uses thereof for detecting an analyte on or within a sample, as defined in the claims.

Unlike common antibodies, which are composed of two heavy chains and two light chains, a single-domain antibody (sdAb) is a single-chain antibody polypeptide consisting of a single monomeric variable antibody domain.

Single-domain antibodies are typically about 110 amino acids long and have a typical molecular weight in the region of 12-15 kDa. As such, single-domain antibodies are much smaller than common antibodies (150-160 kDa), and even smaller than Fab fragments (which consist of one light chain and half a heavy chain and have a molecular weight of about 50 kDa) and single-chain variable fragments (which consist of two variable domains, one from a light and one from a heavy chain, and have a molecular weight of about 25 kDa).

The first single-domain antibodies, called "V_{H}H fragments", were engineered from heavy-chain antibodies found in camelids. Single-domain antibodies called "V_{NAR} fragments" can also be obtained from the heavy-chain antibodies of cartilaginous fishes (Ig_{NAR}, 'immunoglobulin new antigen receptor'). Single-domain antibodies can also be obtained by splitting the dimeric variable domains from common immunoglobulin G (IgG) (eg. from humans or mice) into monomers. Although most research into single-domain antibodies is currently based on heavy-chain variable domains, single-domain antibodies derived from light chains have also been shown to bind specifically to target epitopes.

Single-domain antibodies have been obtained in the art by immunization of dromedaries, camels, Ilamas, alpacas or sharks with the desired antigen and subsequent isolation of the mRNA coding for heavy-chain antibodies. A gene library of single-domain antibodies containing several million clones is produced by reverse transcription and PCR, and screening techniques such as phage display and ribosome display are used to identify clones binding the antigen.

Alternatively, single-domain antibodies have been made in the art from common murine or human IgGs. A problem with this approach is that the binding region of a common IgG consists of two domains (V_{H} and V_{L}), which tend to dimerize or aggregate because of their lipophilicity. Monomerization is usually accomplished by replacing lipophilic by hydrophilic amino acids, but often results in a loss of affinity to the antigen.

Given their simple domain architecture, single-domain antibodies lend themselves to recombinant expression as fusion proteins.

The ease of genetic manipulation of *E*. *coli* makes it an attractive host for expressing antibodies. It is known in the art to express labelled antibody fragments from the periplasm of *E*. *coli.* By way of example, Dooley M.F. et al., Mol. Imm., (2003) 40:25-33; Arbabi M. et al., FEBS Letters (1997) 414:521-526; and Nuttall S.D. et al., Eur. J. Biochem. 2003 Sep. 270(17):3543-54 describe high level expression of camelid and shark antibodies in the *E. coli* periplasm. However, these expression systems are associated with a number of technical problems, such as unreliable yields and inconsistent secretion of active antibody polypeptides.

Low-level cytoplasmic expression of antibodies has also been described (see Bossi S. et al., Protein Expression and Purification, Volume 72, Issue 1, July 2010, pages 55-58), using specialized strains of *E*. *coli* with altered conditions and in the presence of stabilising antigen. The technical challenges associated with cytoplasmic expression of antibodies reflect the requirement for the formation of one or more intra-chain disulfide bonds within the single domain antibody peptide chain, which are essential to constrain the structure of the antibody, hence yielding high affinity binding properties. The proteins involved in forming disulfide bonds and in shuffling these within complex proteins to generate a single preferred conformation are situated within the periplasm of *E*. *coli.* In contrast, the cytoplasm of *E*. *coli* is generally regarded as being a reductive environment, which does not favour the formation of disulfide bonds and actively promotes their reduction to two free sulfhydryl groups. A small number of *E*. *coli* strains have been engineered to provide periplasm-like conditions in the cytoplasm - eg. by generating a non-reducing environment (eg. by knocking out cytoplasmic thioredoxin). However, these strains tend to grow less well than native strains, leading to a reduction in the biomass generated and also give lower expression levels, hence reducing the yield of protein.

In view of these technical problems, cytoplasmically expressed antibody fragments or domains thereof are almost universally mis-folded and unable to bind to their target antigen with high affinity.

There is therefore a need in the art for reliable and efficient means to express single-domain antibodies and antibody fragments in the *E*. *coli* cytoplasm that overcome one or more of the problems encountered in the prior art.

Dooley, H. et al. Molecular Immunology, September 2003, Vol. 40(1), pages 25-33, describes the selection and characterisation of naturally-occurring single-domain (IgNAR) antibody fragments from immunized sharks by phage display.

Ghahroudi M. Arbabi et al., FEBS Letters, 1997, Vol. 414(3), pages 521-526, describes the selection and identification of single domain antibody fragments from camel heavy-chain antibodies.

The present invention meets this need in the art by expressing single-domain antibodies or antibody fragments in the *E*. *coli* cytoplasm as a fusion protein with a multimeric thermostable kinase, as defined in the claims.

Thus, in a first aspect of the invention, there is provided a single-chain fusion protein comprising: (i) a multimeric thermostable kinase; and (ii) a single-domain antibody or single-domain antibody fragment, as defined in the claims.

The invention also provides a method of preparing a single-domain antibody or antibody fragment, as defined in the claims, the method comprising: (i) expressing the single-domain antibody or antibody fragment as a single-chain fusion protein with a multimeric thermostable kinase, in a host cell such as *E*. *coli;* and (ii) purifying the fusion protein from the cytoplasm of the host cell.

It is known in the art to express and purify thermostable kinases as recombinant proteins from the cytoplasm of *E*. *coli,* providing more accessible and reproducible material than when isolated from the host thermophilic organisms (see WO 2005/093085, WO 2009/104013 and WO 2010/079357). As thermostable adenylate kinases (tAKs), such as those from *Sulfolobus acidocaldarius* and *Thermotoga maritima,* lack intramolecular disulfide bonds, these enzymes have been shown to fold correctly within the relatively oxidising environment of the *E*. *coli* cytoplasm.

Thermostable proteins have become increasingly used as fusion tags. In this regard, fusion of proteins which are intrinsically soluble even at high temperatures is known to promote folding of passenger proteins [de Marco et al., J. Biotech., (2004) 107:125-133; Fox J.D. et al., FEBS Lett. 2003 Feb 27;537(1-3):53-7; Kondo N. et al., Anal Biochem. 2009 Feb 15;385(2):278-85. Epub 2008 Nov 19; Luke J.M. et al., J Biotechnol. 2011 Feb 10;151(3):242-50. Epub 2010 Dec 17.] This approach has not previously been used for the expression of recombinant antibodies, due to problems with obtaining the correct disulfide bond formation and the need for separate fusion tags on each antibody chain, mainly due to the cytoplasmic localisation of these fusion partners.

The present inventors have unexpectedly identified that, when expressed from the cytoplasm, the multimeric thermostable kinase component of the fusion protein actively promotes the folding of the single-domain antibody component and provides a stabilizing function, whilst retaining measurable kinase activity.

Furthermore, the fusion proteins of the invention can be purified from the cytoplasm of *E*. *coli* in active form with yields higher than would be expected for the same single-domain antibody or antibody fragment in the periplasm.

The conference of high-level thermal stability onto a single-domain antibody fragment has not been described previously. In this context "high level thermal stability" refers to thermal stability above 80°C, preferably above 90°C.

The high stability of the expressed fusion proteins advantageously allows their purification via a rapid protocol involving high temperature treatment of *E*. *coli* cell lysate, which is clarified and applied to a single chromatography column. The high stability of the expressed fusion proteins also advantageously allows for their storage at room temperature (rather than at 4°C or below).

The high stability of the fusion protein of the invention is also advantageous if the fusion protein is used as a reporter molecule in detection assays. By way of example, the use of a highly stable fusion protein of the invention allows the addition of high levels of urea to the sample extractant or diluents, enabling improved antigen extraction and background reduction (as detailed in WO2010/079357) with no loss of detection sensitivity. Treatment of the sample with urea also allows the detection of different types of antigen, focussing on conserved epitopes which would not normally be solvent exposed.

As used herein, the term "single-domain antibody" embraces any single-chain antibody polypeptide consisting of a single monomeric variable antibody domain. Examples of single-domain antibodies include "V_{H}H fragments" (eg. engineered from camelid heavy-chain antibodies) and "V_{NAR} fragments" (eg. obtained from an Ig_{NAR} heavy-chain antibody of cartilaginous fishes, such as shark).

In one embodiment, the single-domain antibody or antibody fragment is a V_{NAR} fragment of a shark immunoglobulin new antigen receptor (Ig_{NAR}). One example of a V_{NAR} sequence of a single-domain antibody from shark is provided herein as SEQ ID NO: 8.

In one embodiment, the single-domain antibody or antibody fragment is a V_{H}H fragment of a camelid heavy-chain antibody. One example of a V_{H}H sequence of a single-domain antibody from camelid is provided herein as SEQ ID NO: 13.

The single-domain antibody component of the fusion protein binds an antigen.

As used herein, the term "single-domain antibody fragment" embraces a fragment of any "single-chain antibody" as defined herein. Examples of fragments include truncated genes that encode only the complementarity determining region (CDR), responsible for determining the antigen specificity; in the case of V_{NAR} fragments only CDR3, possibly constrained artificially by one or more conventional techniques known to those familiar in the art. Antibody fragments of the invention may be produced by recombinant DNA techniques. In one embodiment, the single-domain antibody fragment is at least 50 amino acids long, such as at least 60, 70, 80, 90 or 100 amino acids long.

The single-domain antibody fragment component of the fusion protein binds an antigen. A fragment of a single-domain antibody is capable of binding to the same antigen to which the full-length single-domain antibody binds.

Binding of the single-domain antibody or single-domain antibody fragment to the antigen (to form an antibody-antigen complex) can be detected using any conventional assay known in the art, and may form the basis of an assay for detecting an analyte comprising the antigen.

There are a number of technical advantages associated with the use of single-domain antibodies (or fragments thereof), as compared with conventional 'whole antibodies'. By way of example, the comparatively low molecular mass of single-domain antibodies leads to good permeability in tissues, and a short plasma half-life. Furthermore, as they lack an Fc region, single-domain antibodies do not induce complement-triggered cytotoxicity. Some single-domain antibodies and antibody fragments bind to 'hidden' antigens (eg. the active sites of enzymes) that are not accessible to whole antibodies; this property has been shown to result from their extended CDR3 loop, which is able to penetrate such sites.

Single-domain antibodies and antibody fragments thereof therefore lend themselves to the development of novel diagnostic reagents and therapeutics.

In one embodiment, demonstrated in the Examples and Figures, an exemplary single-domain antibody is derived from either camelid heavy-chain antibody (V_{H}H) or the variable domain of shark Ig new antigen receptor (V_{NAR}).

As used herein, the term "antibody component" refers to the single-domain antibody and single-domain antibody fragment as defined herein.

Any kinase enzyme may be used in the present invention, so long as it is "thermostable" and "multimeric".

The term "thermostable kinase" refers to a kinase that retains kinase activity after exposure to heat - ie. that is relatively unaffected by high temperatures. In one embodiment of the invention, the kinase activity of a thermostable kinase after exposure to a temperature of between 50-120°C is at least 70% (or at least 80%, 90%, 95% or 100%) of the kinase activity of the kinase prior to the exposure. In one embodiment, after exposure to 40°C for 30 minutes, or after exposure to 50°C for 30 minutes, or after exposure to 60°C for 30 minutes, or after exposure to 70°C for 30 minutes, or after exposure to 80°C for 20 minutes, or after exposure to 90°C for 10 minutes, or after exposure to 120°C for 3 minutes, a thermostable kinase may retain at least 70% of the pre-exposure kinase activity (or at least 80%, 90%, 95% or 100% of the pre-exposure kinase activity).

Thermostable kinases may also be more resistant than non-thermostable kinases to a range of other biochemical and physical processes that routinely damage or destroy proteins or render them inactive, such as exposure to certain chemicals eg. chaotropes, free-radical damage, detergents, extremes of pH, exposure to proteases, protein cross-linking, encapsulation within non-permeable or semipermeable membranes or polymers, or irreversible immobilisation onto surfaces (see for example: Daniel R.M. et al., Biochem J. 1982 207:641-4; Rees DC and Robertson AD, Protein Sci. 2001 10:1187-94; Burdette DS et al., Enzyme Microb Technol. 2000 27:11-18; Scandurra R. et al., Biochimie. 1998 Nov;80(11):933-41; and Liao HH. Enzyme Microb Technol. 1993 Apr;15(4):286-92). In one embodiment, after exposure to one or more of the biochemical and physical processes described above, thermostable kinases may retain at least 70% (or 80%, 90%, 95% or 100%) of their pre-exposure kinase activity.

It is a matter of routine for a skilled person to identify whether a kinase is "thermostable", by measuring the "retained kinase activity" using any of the conventional tests available in the prior art.

A skilled person is familiar with a wide range of different thermostable kinases suitable for use in the present invention.

Thermostable kinases have a variety of recognized tertiary structures, and can be broadly classified into two groups based on their molecular architecture - ie. "multimeric"/ "polymeric" kinases or "monomeric" kinases.

The thermostable kinase forms multimers - ie. the thermostable kinase is a "multimeric" kinase, such a dimeric, trimeric, tetrameric, pentameric, hexameric, heptameric or octameric kinase. Multimeric (eg. dimeric, trimeric, tetrameric, hexameric, octameric) tertiary structures may be associated with an improved stability of the kinase to conditions such as temperature, pH, chemical denaturants, or proteases.

In one embodiment, the thermostable kinase forms trimers - ie. the thermostable kinase is a "trimeric" thermostable kinase.

By way of example, the thermostable kinase enzymes from *Sulfolobus* species (eg. *S*. *acidocaldarius*) have a trimeric structure with a central hydrophobic core that is the principle determinant in maintaining their activity at high temperatures. An example of a trimeric thermostable kinase is provided in SEQ ID NO: 6 (adenylate kinase from *S*. *acidocaldarius*).

In one embodiment, the thermostable kinase is a dimeric thermostable kinase. Acetate kinases, such as the enzyme from *Methanosarcina thermophila* (SEQ ID NO: 1 provided herein) and *Thermotoga maritima* (SEQ ID NO: 19), are dimeric.

In one embodiment, the thermostable kinase is a tetrameric thermostable kinase. Pyruvate kinases, such as the enzyme from *Geobacillus stearothermophilus* (SEQ ID NO: 2 provided herein), are tetrameric.

In one embodiment, the thermostable kinase is a hexameric thermostable kinase. Uridine monophosphate (UMP) kinases, such as the enzyme from *Pyrococcus furiosus* (SEQ ID NO: 3 provided herein), *Sulfolobus acidocaldarius* (SEQ ID NO: 21) and *Thermotoga maritima* (SEQ ID NO: 20), are hexameric.

In one embodiment, the thermostable kinase is an octameric thermostable kinase. Butyrate kinases, such as those from *Thermotoga maritima* (SEQ ID NO: 4 provided herein), are octameric.

Other thermostable kinase enzymes have a monomeric structure, exemplified by the adenylate kinases from *Thermatoga* species (eg. *T. maritima,* see SEQ ID NO: 5 provided herein). These kinases have a slightly longer polypeptide chain with an additional "lid" domain that affects the active site.

In one embodiment, the thermostable kinase is an adenylate kinase, acetate kinase, UMP kinase, pyruvate kinase or butyrate kinase. In one embodiment, the thermostable kinase is a trimeric adenylate kinase. In one embodiment, the thermostable kinase is a dimeric acetate kinase. In one embodiment, the thermostable kinase is a hexameric UMP kinase.

In one embodiment, the thermostable kinase is a microbial kinase of an organism selected from the group consisting of *Pyrococcus furiosus, P.abyssi, P. horikoshii, P. woesii;* a *Sulfolobus sp. such as Sulfolobus solfataricus, S.acidocaldarius, or S. shibatael; a Thermotoga sp.* such as *Thermatoga maritima* or *T. neapolitana;* a *Methanococcus spp.; Rhodothermus marinus, Thermococcus litoralis, Geobacillus stearothermophilus,* and *Methanosarcina thermophila.*

In one embodiment, the thermostable kinase may be an *A. fulgidus* kinase, *A*. *pernix* kinase, *A. pyrophilus* kinase, *B*. *caldotenax* BT1 kinase, *Bacillus species* PS3 kinase, *B*. *stearothermophilus* 11057 kinase, *B. stearothermophilus* 12001 kinase, *B*. *thermocatenulatus* kinase, *C*. *stercocorarium* kinase, a *G*. *stearothermophilus* kinase, *Methanococcus spp.* kinase, *M. thermophila* kinase, *M. ruber* kinase, *P*. *abyssi* kinase, *P. furiosus* kinase, *P. horikoshii* kinase, *P*. *woesii* kinase, *R. marinus* kinase, *S*. *acidocaldarius* kinase, S. *shibatae* kinase, *S*. *solfataricus* kinase, *T. ethanolicus* kinase, *T. thermosulfurogenes* kinase, *T. celere* kinase, *T. litoralis* kinase, *T. aquaticus* YT1 kinase, *T. caldophilus* GK24 kinase, *T. thermophilus* HB8 kinase, *T. maritima* kinase, *or a T. neapolitana* kinase.

In one embodiment, the thermostable kinase is a *T. maritima* dimeric acetate kinase. In one embodiment, the thermostable kinase is a *S*. *acidocaldarius* trimeric adenylate kinase. In one embodiment, the thermostable kinase is a *S*. *acidocaldarius* hexameric UMP kinase. In one embodiment, the thermostable kinase is a *T. maritima* hexameric UMP kinase.

In one embodiment, the term "thermostable kinase" embraces sequence variants of any of the thermostable kinases of the invention discussed herein, and also embraces fragments thereof (including fragments of the sequence variants, and sequence variants of the fragments).

In this regard, the genetic modification of thermostable kinases has been shown to provide significant increases in thermal stability. A comparison of the thermostability of a range of kinase enzymes with the defined 3-D structure of the trimeric (archaeal) AKs has identified amino acids that influence stability (Vonrhein et al. (1998) J. Mol. Biol. 282:167-179 and Criswell et al. (2003) J. Mol. Biol.330:1087-1099)

Genetically engineered variants of thermostable kinases showing modified (eg. improved) thermostability may be identified via the specific site-directed mutagenesis of amino acids (eg. amino acids believed to form part of the central core packing region of the trimeric molecule) or by random "directed evolution" methods where the whole kinase molecule is subjected to subsequent rounds of mutagenesis and selection/ screening for molecules with improved properties.

In one embodiment, kinase activity of the "thermostable kinase" polypeptide is not required. In accordance with this embodiment, the sole purpose of the "thermostable kinase" component of the fusion protein is to facilitate (eg. increase) production of the single-domain antibody component. In accordance with this embodiment, the term "thermostable kinase" embraces sequence variants and truncated polypeptides that have a modified (eg. reduced) kinase activity as compared with the kinase activity of the reference kinase. By way of example, the variant or truncated polypeptide may have substantially no kinase activity.

In one embodiment, the thermostable kinase comprises or consists of an amino acid sequence having at least 70%, 80%, 85%, 90%, 95%, 99% or 100% sequence identity to the amino acid sequence of any of SEQ ID NOs: 1-6, or a fragment thereof comprising at least 50, 75, 100, 125, 150, 175, 200, 225, 250, 275, 300, 325, 350, 375, 400, 425, 450, 475, 500, 525 or 550 consecutive amino acids thereof.

In one embodiment, the thermostable kinase comprises or consists of an amino acid sequence comprising at least 50, 75, 100, 125, 150, 175, 200, 225, 250, 275, 300, 325, 350, 375, 400, 425, 450, 475, 500, 525 or 550 consecutive amino acids of any of SEQ ID NOs: 1-6, or an amino acid sequence having at least 70%, 80%, 85%, 90%, 95%, 99% or 100% sequence identity thereto.

Other examples of suitable reporter kinases are disclosed in WO 00/46357 and WO 2005/093085.

As used herein, the term "thermostable kinase component" refers to the thermostable kinase (including variants/ fragments thereof) as defined herein.

The fusion protein of the invention comprises both (i) a multimeric thermostable kinase component; and (ii) a single-domain antibody or single-domain antibody fragment component, as defined in the claims.

The thermostable kinase and antibody components of the fusion protein can be fused in any order. Thus, in one embodiment, the thermostable kinase is expressed N-terminally to the single-domain antibody/ antibody fragment in the fusion protein (ie. N-[thermostable kinase]-[antibody component]-C). In an alternative embodiment, the single-domain antibody/ antibody fragment of the fusion protein is expressed N-terminally to the thermostable kinase in the fusion protein (ie. N-[antibody component]-[thermostable kinase]-C).

In one embodiment, the fusion protein of the invention comprises multiple (ie. at least 2 or more, such as 3, 4, 5, 6, 7 or 8) single-domain antibodies and/ or antibody fragments, which may be the same or different.

By way of example, the fusion protein may comprise a thermostable kinase and two or more single-domain antibodies or antibody fragments, which may be the same, or may be different. For example, each thermostable kinase may be fused to multiple single-domain antibodies and/ or antibody fragments, which may be the same or different. For example, the fusion protein may comprise a single-domain antibody or antibody fragment fused at the N-terminus of the thermostable kinase, and another single-domain antibody or antibody fragment fused at the C-terminus of the thermostable kinase, where the N-terminal single-domain antibody/ antibody fragment and the C-terminal single-domain antibody/ antibody fragment may be the same, or may be different.

As such, in one embodiment, the order of the components within the fusion protein is:
N-[antibody component 1]-[thermostable kinase]-[antibody component 2]-C (where antibody component 1 and 2 can be the same or different).

The different single-domain antibodies/ antibody fragments within the fusion protein may differ from each other in terms of their binding activity. In this regard, the different single-domain antibodies or antibody fragments fused to the same thermostable kinase may bind to different analytes, or may bind to different epitopes of the same analyte, or may bind to the same analyte/ epitope but with different affinities. In one embodiment, the different analytes bound by the single-domain antibodies or antibody fragments may be (or be derived from/ components of) different biological infectious or non-infectious agents (eg. different bacterial or viral antigens). In one embodiment, the different analytes bound by the single-domain antibodies or antibody fragments may be a combination of (i) analytes from one or more different biological infectious/ non-infectious agents and (ii) analytes having a detection function ("detector molecules".

In one embodiment, the fusion protein of the invention comprises multiple (ie. at least 2 or more, such as 3, 4, 5, 6, 7 or 8) thermostable kinases, which may be the same or different.

By way of example, the fusion protein may comprise a single-domain antibody or antibody fragment and two or more thermostable kinases, which may be the same, or may be different. For example, each single-domain antibody or antibody fragment may be fused to multiple (eg. 2, 3, 4, 5, 6, 7 or 8) thermostable kinases, which may be the same or different. For example, the fusion protein may comprise a thermostable kinase fused at the N-terminus of the single-domain antibody or antibody fragment, and another thermostable kinase fused at the C-terminus of the single-domain antibody or antibody fragment, where the N-terminal thermostable kinase and the C-terminal thermostable kinase may be the same, or may be different.

As such, in one embodiment, the order of the components within the fusion protein is:
N-[thermostable kinase 1]-[antibody component]-[thermostable kinase 2]-C where thermostable kinase 1 and 2 can be the same or different.

The different thermostable kinases may differ from each other in terms of (for example) their target, and/ or in terms of their level of kinase activity.

In one embodiment, the molecular weight of the single-domain antibody or antibody fragment component is over 50% of the molecular weight of the thermostable kinase (such as over 60%, 70%, 80% or 90% of the molecular weight of the thermostable kinase). In one embodiment, the total molecular weight of the single-domain antibody or antibody fragment component is greater than the molecular weight of the thermostable kinase. In one embodiment, the total molecular weight of the single-domain antibody or antibody fragment component forms over 50% of the molecular weight of the fusion protein (such as over 60%, 70%, 80% or 90% of the molecular weight of the fusion protein).

The fusion protein of the invention may be prepared using conventional techniques (eg. as described in Examples 1, 2, 6 and 7), such as by fusing a nucleic acid sequence encoding a thermostable kinase (as defined above) and a nucleic acid sequence encoding a single-domain antibody or antibody fragment (as defined above).

The present invention thus provides a polynucleotide sequence encoding a fusion protein of the invention as defined in the claims.

The nucleic acid sequences encoding the thermostable kinase and antibody components of the fusion protein can be arranged/ fused in any order. Thus, in one embodiment, the nucleic acid sequence encoding the thermostable kinase may be located/ fused 5' to the nucleic acid sequence encoding the single-domain antibody or antibody fragment. Alternatively, the nucleic acid sequence encoding the single-domain antibody or antibody fragment may be located/ fused 5' to the nucleic acid sequence encoding the thermostable kinase.

In one embodiment, the polynucleotide sequence encoding the fusion protein of the invention comprises multiple (ie. at least 2 or more) nucleic acid sequences encoding single-domain antibodies and/ or antibody fragments, which may be the same or different (eg. as discussed above).

Alternatively, or in addition, the polynucleotide sequence encoding the fusion protein of the invention may comprise multiple (ie. at least 2 or more) nucleic acid sequences encoding thermostable kinases, which may be the same or different (eg. as discussed above).

In one embodiment, the polynucleotide sequence encodes a fusion protein as discussed above, comprising a thermostable kinase and two single-domain antibodies or antibody fragments, which may be the same or different. In accordance with this embodiment, the polynucleotide sequence encoding the fusion protein comprises a nucleic acid sequence encoding a thermostable kinase and nucleic acid sequences encoding two single-domain antibodies or antibody fragments. For example, the polynucleotide sequence may comprise the following sequences in a 5' to 3' direction: a nucleic acid sequence that encodes a first single-domain antibody or antibody fragment, a nucleic acid sequence that encodes a thermostable kinase, and a nucleic acid sequence that encodes a second single-domain antibody or antibody fragment, wherein the first and second single-domain antibodies/ fragments are the same or different.

In one embodiment, the polynucleotide sequence encodes a fusion protein as discussed above, comprising a single-domain antibody or antibody fragment and two thermostable kinases. In accordance with this embodiment, the polynucleotide sequence encoding the fusion protein comprises a nucleic acid sequence encoding a single-domain antibody or antibody fragment and nucleic acid sequences encoding two thermostable kinases. For example, the polynucleotide sequence may comprise the following sequences in a 5' to 3' direction: a nucleic acid sequence that encodes a first thermostable kinase, a nucleic acid sequence that encodes a single-domain antibody or antibody fragment, and a nucleic acid sequence that encodes a second thermostable kinase, wherein the first and second thermostable kinases are the same or different.

The polynucleotide sequence may optionally comprise one or more linker regions located between the coding sequences for the thermostable kinase and single-domain antibody components of the fusion protein.

As such, the polynucleotide sequence may comprise the following components in any of the following orders:
5'-[thermostable kinase coding sequence]-[linker]-[antibody coding sequence]-3'
5'-[antibody coding sequence]-[linker]-[thermostable kinase coding sequence]-3'
5'-[antibody coding sequence]-[linker]-[thermostable kinase coding sequence]-[linker]-[antibody coding sequence]-3'
5'-[thermostable kinase coding sequence]-[linker]-[antibody coding sequence]-[linker]-[thermostable kinase coding sequence]-3'

Examples of suitable conventional linker sequences known in the art include unstructured linkers (eg. (GGGGS)n where n=3-5) or helical linkers (eg. LAEAAAKEAAAKEAAAKEAAAKAAA).

The polynucleotide sequence may optionally comprise one or more sequences encoding a cleavage site, which may be located between the coding sequences for the thermostable kinase and single-domain antibody components of the fusion protein. The encoded cleavage site may be recognised by a protease. This allows separation of the thermostable kinase and the single-domain antibody or antibody fragment, if required, by cleavage at the cleavage site. For example, the thermostable kinase and single domain antibody components may be separated by a cleavage site recognised by factor Xa (IEGR↓), enterokinase (DDDDK↓), or TEV protease (ENLYFQ↓G). Other examples of cleavage sites are described in the literature.

Optionally the components of the fusion protein may be separated by the inclusion of an intein cleavage site between the thermostable kinase and the antibody or antibody fragment. This may require the additional presence of an intein domain at the C-terminus of the fusion or positioned sequentially to the thermostable kinase domain. Hence, in accordance with this embodiment, the fusion protein may take one of the following configurations:
N-Thermostable kinase-intein domain-intein cleavage site-Antibody component-C or
N-Thermostable kinase-intein cleavage site-Antibody component-intein domain-C

The present invention also provides a plasmid comprising a polynucleotide sequence encoding a fusion protein of the invention as defined in the claims.

The plasmid may comprise one or more additional polynucleotide sequences, which may be operably linked in frame to the polynucleotide sequence encoding the fusion protein. By way of example, the plasmid may comprise a polynucleotide sequence encoding a polypeptide tag or label, and expression from the plasmid may produce a tagged/ labelled fusion protein. Optionally, any additional polynucleotide sequence may be separated from the polynucleotide sequence encoding the fusion protein by a linker sequence and/ or by a sequence encoding a cleavage site, as discussed above. The presence of a cleavage site allows the fusion protein to be separated from any additional polypeptide (eg. tag or label) encoded by the additional polynucleotide sequence.

Gene expression can be driven from any of the described promoter systems familiar to those with knowledge in the art, such as IPTG-inducible lacZ based promoters or tet promoters. Expression may for example be driven by the malate dehydrogenase promoter (MDH) as described previously (Ungurs et al., J. Hospital Infection, Vol. 74, 2010, pages 144-151 "Quantitative measurement of the efficacy of protein removal by cleaning formulations; comparative evaluation of prion-directed cleaning chemistries").

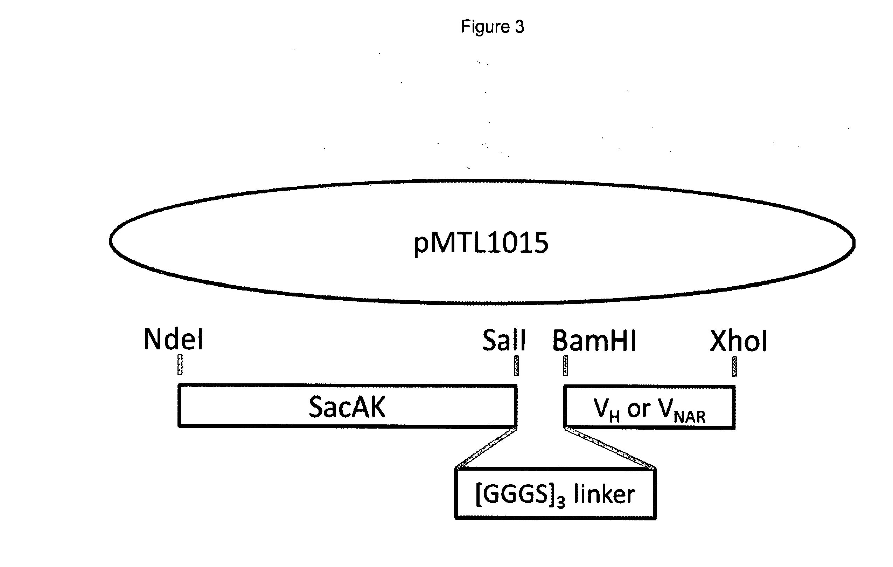

Conventional, commercially available expression systems can be used, typically a Gateway-adapted vector such as a pMTL1015 vector (Ungurs *et al.* 2010, as above). The Gateway-adapted vector illustrated in Figure 3 has a cassette-based method of construction and can be used to rapidly generate alternative single domain antibody fusions. Other cassette-based vectors can also be used to construct the single domain antibody fusion proteins described herein.

The polynucleotide sequence can be expressed in the cytoplasm of a host cell using conventional techniques in the art.

Suitable host cells include prokaryotic and eukaryotic cells, preferably prokaryotic host cells such as *E*. *coli.* In one embodiment, recombination-defective host cells may be used, in order to minimise re-arrangement of the gene constructs during expression (eg. when two or more different fusion proteins are expressed in the same host cell). By way of example, a suitable recombination-defective host cell that may be used to express the fusion proteins of the invention is *E*. *coli* RV308.

Thus, in one aspect, the invention provides a method (as defined in the claims) of producing a fusion protein of the invention as defined in the claims, comprising expressing a polynucleotide sequence encoding the fusion protein, or a plasmid comprising said polynucleotide sequence, in a host cell such as *E*. *coli,* and purifying the expressed fusion protein from the cytoplasm of the host cell.

In one embodiment of the invention, multiple different fusion proteins (which may be the same or different) are expressed in a single host cell. These fusion proteins can be expressed from a single plasmid using the same or different promoters. Alternatively, the fusion proteins can be expressed from two different compatible plasmids. The ratio of the fusion proteins expressed from the plasmid or plasmids may be varied by using high or low copy number plasmids, and/or high, medium or low expressing promoters (eg. as illustrated in Example 13 part (ii)). As discussed below, the expressed fusion proteins may multimerise to form a multimeric protein complex, which can be recovered from the host cell.

The invention also provides a method (as defined in the claims) of preparing a single-domain antibody or single-domain antibody fragment as defined in the claims, the method comprising:
(i) expressing the single-domain antibody or antibody fragment as a single-chain fusion protein with a multimeric thermostable kinase, in a host cell such as *E*. *coli*; and
(ii) purifying the fusion protein from the cytoplasm of the host cell.

All embodiments of the fusion proteins of the invention (as defined herein) apply equally to the corresponding polynucleotides (and plasmids, host cells etc.), and to the expression methods of the invention.

The expressed fusion protein may be extracted from the cytoplasm of the host cell (eg. *E*. *coli*) by any conventional technique known in the art. Typical extraction protocols may comprise lysing the host cells, and separating the soluble portion of the lysate by centrifugation.

The expressed, extracted fusion protein may be purified from the host cell lysate by any conventional technique known in the art.

In one embodiment, in view of the heat stability of the expression fusion protein, the purification step may involve high temperature treatment of the lysate (eg. at approx 80°C for about 20 or 30 minutes). Following heat treatment, lysate may be clarified by removal of precipitated proteins. The purification step may comprise the step of applying the lysate to a chromatography column (eg. a Blue Sepharose column) and eluting the fusion protein using AMP and ATP.

Fusion proteins may be optionally further purified using an affinity column loaded with the ligand(s) for the single-domain antibody component(s). This step may be particularly useful to ensure that fusion proteins (or multimeric fusion protein complexes) comprising two or more different antibody components binding different ligands do indeed contain each of the different antibody components.

Functional recovery of correctly folded single-domain antibody or antibody fragment can be assessed in a conventional ELISA assay, using the thermostable kinase activity (eg. tAK activity) as the assay read-out.

As discussed above, the thermostable kinases used in the invention are known to form multimeric complexes.

As such, in one embodiment, the fusion proteins of the invention are recovered from the host cell as multimers (a multimeric fusion protein complex).

Formation of a multimeric fusion protein complex is due to multimerisation between the thermostable kinase components of multiple (ie. 2 or more, such as 2, 3, 4, 5, 6, 7 or 8) fusion proteins of the invention. By way of example, formation of a trimeric fusion protein complex is due to trimerisation between the thermostable kinase components (monomers) of 3 fusion proteins of the invention.

In one embodiment, the fusion proteins of the invention form multimers following recovery from the host cell (eg. during purification and refolding). The presence of high salt concentrations in the buffer used during the lysis phase may assist in the recovery of correctly folded proteins. Thus, in one embodiment, the lysis is carried out in a buffer solution containing 900mM, 1000mM, 1200mM or 1500mM NaCl, 500mM, 700mM 900mM NH₄SO₄, or equivalents. The addition of organic solvents may also preferentially promote correct folding in the recovered proteins.

Thus, the invention provides a multimeric fusion protein complex comprising multiple (ie. 2 or more, such as 2, 3, 4, 5, 6, 7 or 8) fusion proteins of the invention, as defined in the claims.

The terms "multimer"/ "multimeric" and the terms "polymer"/ "polymeric" are used interchangeably herein and embrace, for example, dimers, trimers, pentamers, tetramers, hexamers, heptamers and octamers.

In one embodiment, a multimeric fusion protein complex of the invention is a dimeric fusion protein complex comprising two fusion proteins of the invention; or a trimeric fusion protein complex comprising three fusion proteins of the invention; or a tetrameric fusion protein complex comprising four fusion proteins of the invention; or a hexameric fusion protein complex comprising four fusion proteins of the invention; or an octomeric fusion protein complex comprising four fusion proteins of the invention.

A multimeric fusion protein complex of the invention thus comprises a thermostable kinase multimer (formed by multimerisation of thermostable kinase components of multiple fusion proteins of the invention), wherein each individual monomer of the thermostable kinase multimer is fused to a (or at least one) single-domain antibody or antibody fragment. In one embodiment of the present invention, each individual monomer of the thermostable kinase multimer may be fused to two or more single-domain antibodies or antibody fragments, which may be the same, or may be different (as discussed above).

By way of example, the multimeric fusion protein complex may comprise a thermostable kinase dimer, wherein each of the two thermostable kinase monomers of the dimer is fused to a single-domain antibody or antibody fragment. The fusion protein may comprise a thermostable kinase trimer, wherein each of the three thermostable kinase monomers is fused to a single-domain antibody or antibody fragment. The fusion protein may comprise a thermostable kinase tetramer, wherein each of the four thermostable kinase monomers is fused to a single-domain antibody or antibody fragment. The fusion protein may comprise a thermostable kinase hexamer, wherein each of the six thermostable kinase monomers is fused to a single-domain antibody or antibody fragment. The fusion protein may comprise a thermostable kinase octomer, wherein each of the eight thermostable kinase monomers is fused to a single-domain antibody or antibody fragment.

Alternatively, the multimeric fusion protein complex may comprise a thermostable kinase dimer, wherein each of the two thermostable kinase monomers of the dimer is fused to two or more single-domain antibodies or antibody fragments, which may be the same, or may be different. The fusion protein may comprise a thermostable kinase trimer, wherein each of the three thermostable kinase monomers is fused two or more single-domain antibodies or antibody fragments, which may be the same, or may be different. The fusion protein may comprise a thermostable kinase tetramer, wherein each of the four thermostable kinase monomers is fused to two or more single-domain antibodies or antibody fragments, which may be the same, or may be different. The fusion protein may comprise a thermostable kinase hexamer, wherein each of the six thermostable kinase monomers is fused to two or more single-domain antibodies or antibody fragments, which may be the same, or may be different. The fusion protein may comprise a thermostable kinase octomer, wherein each of the eight thermostable kinase monomers is fused to two or more single-domain antibodies or antibody fragments, which may be the same, or may be different.

The fusion proteins of the invention that make up the multimeric fusion protein complex of the invention may be the same or different (ie. the multimeric complexes may be homo-multimeric or hetero-multimeric). For example, the multimeric fusion protein complex of the invention may be homo-dimeric, homo-trimeric, homo-tetrameric, homo-hexameric or homo-octomeric. Alternatively, the multimeric fusion protein complex of the invention may be hetero-dimeric, hetero-trimeric, hetero-tetrameric, hetero-hexameric or hetero-octomeric.

In one embodiment, the single-domain antibodies or antibody fragments within the multimeric fusion protein complex are all the same. Thus, the same single-domain antibodies or antibody fragments are fused to each of the individual thermostable kinase monomers.

In an alternative embodiment, the single-domain antibodies or antibody fragments that are fused to the individual monomers of the thermostable kinase within the multimeric complex are not all the same. In accordance with this embodiment, the multimeric fusion protein complex comprises a mixture of two or more different single-domain antibodies/ antibody fragments (eg. 2, 3, 4, 5, 6, 7 or 8 different single-domain antibodies/ antibody fragments), each fused to individual thermostable kinase monomers. By way of example, the different antibodies/ antibody fragments may be located within the same fusion protein, or in different fusion proteins.

The different single-domain antibodies/ antibody fragments may differ from each other in terms of their binding activity. In this regard, the different single-domain antibodies or antibody fragments fused to different thermostable kinase monomers may bind to different analytes, or may bind to the different epitopes of the same analyte, or may bind to the same analyte/ epitope but with different affinities. In one embodiment, the different analytes bound by the single-domain antibodies or antibody fragments may be (or be derived from/ components of) different biological infectious or non-infectious agents (eg. different bacterial or viral antigens). In one embodiment, the different analytes bound by the single-domain antibodies or antibody fragments may be a combination of (i) analytes from one or more different biological infectious/ non-infectious agents and (ii) analytes having a detector molecule function.

In one embodiment, all the fusion proteins within the multimeric fusion protein complex are the same (ie. a homo-multimeric fusion protein complex is formed).

In an alternative embodiment, the fusion proteins within the multimeric fusion protein complex are not all the same (ie. a hetero-multimeric fusion protein complex is formed). In accordance with this embodiment, the multimeric fusion protein complex comprises a mixture of two or more different fusion proteins (eg. 2, 3, 4, 5, 6, 7 or 8 different fusion proteins). The different fusion proteins may be expressed either from one plasmid using the same promoter or from one plasmid using different promoters. Alternatively, the different fusion proteins may be expressed from two different (compatible) plasmids.

The ratio of different fusion proteins present in the hetero-multimeric fusion protein complex of the invention will depend on the relative abundance of fusion proteins expressed, which may be varied by using high or low copy number plasmids, and/or high, medium or low expressing promoters (eg. as discussed in Example 13 part (ii)).

All embodiments of the fusion proteins of the invention (as defined herein) apply equally to multimeric complexes thereof, and to the extraction and purification methods of the invention.

In one embodiment, the fusion protein of the invention may be detected by detecting the kinase activity of the thermostable kinase.

Generally, the thermostable kinase is detected using a substrate comprising ADP. The kinase activity of the thermostable kinase converts the ADP to ATP, which is itself used to generate light (eg. by reaction with a bioluminescent reagent).

The term "bioluminescent reagent" refers to any substance or mixture of substances able to react with ATP to generate light, such as a mixture of luciferin and luciferase. Standard luciferin-luciferase assay methods can detect as little as 10⁻¹⁵ moles of ATP. By coupling an enzymatic amplification to the bioluminescent detection methods it is possible to detect as few as 10⁻²⁰ moles of kinase. The light emitted by the reaction of ATP with the bioluminescent reagent can be measured using conventional techniques known in the art, such as using a standard luminometer (eg. a Berthold Orion 96-well microplate luminometer, or a hand-held luminometer).

By way of example, the thermostable kinase can be reacted with ADP at a temperature of between 30°C and 70°C, and the formation of ATP can be detected and measured by bioluminescent detection using luciferin/ luciferase and a suitable luminometer at 20-30°C for 10 minutes to 1 hour.

The data generated by luminometers is expressed in "Relative Light Units" (RLUs). RLUs are a relative, not absolute, measurement. To address this issue, manufacturers have generated data for RLU "factors", which allow the data generated by a given luminometer to be normalised to a calibrated standard. Thus, comparisons can be made between different instruments. The figures given in the present specification relate to measurements taken using a Berthold Orion 96-well microplate luminometer with injector system using a "flash" method of light measurement for 2 seconds immediately after the addition of the luciferase/luciferin reagents (technical specification photomultiplier measuring light emitted at a wavelength of 300-650nm). The RLU factor for the Berthold Orion 96-well microplate luminometer is 1. Accordingly, the RLU values given in the specification can be regarded as standardised/normalised RLU values.

In terms of absolute values, an RLU value can be related to the concentration of ATP required to give said value with the reagents as described in the method. As an approximate conversion, and given the linear relationship between RLU values and ATP concentration, the following values can be used:

| **RLU** | **Approximate concentration of ATP/ µM** |
|---|---|
| 12,000,000 | 1000 |
| 1,200,000 | 100 |
| 120,000 | 10 |
| 12,000 | 1 |
| 1,200 | 0.1 |
| 120 | 0.01 |

In one embodiment, the thermostable kinase component of the fusion protein of the invention has an activity of at least 500,000 RLU per mg kinase, or at least 1,000,000 RLU per mg kinase, or at least 3,000,000 per mg kinase, or at least 5,000,000 RLU per mg kinase, or at least 8,000,000 RLU per mg kinase, or at least 10,000,000 Relative Light Units (RLU) per mg kinase, when measured in the presence of luciferin/luciferase by a luminometer.

Those familiar with the art will recognise other methods for the detection of ATP as the product of a thermostable kinase reaction. These might include detection using absorbance measurement via HPLC, or chemiluminescent, fluorescent or colorimetric measurement using suitable reactive compounds.

In one embodiment of the present invention, the sensitivity of the ATP detection reaction may be further increased by providing one or more additional single-domain antibodies or antibody fragments that bind and recruit one or more additional thermostable kinases to the antibody-analyte complexes (ie. in addition to the antibody and thermostable kinase component(s) present in the fusion protein). Examples of this embodiment of the invention are presented in Examples 12 and 13. The additional single domain antibody/ antibody fragment may be present in the fusion protein targeting the analyte of interest (ie. the analyte being detected). Alternatively, the additional single domain antibody/ antibody fragment may be present in a different fusion protein from the fusion protein targeting the analyte(s) of interest. The fusion protein comprising the single domain antibody/ antibody fragment that binds and recruits an additional thermostable kinase can be present in the form of a single fusion protein, or as part of a homo-multimeric or hetero-multimeric fusion protein complex.

Optionally, the fusion protein that targets the analyte(s) of interest and the fusion protein that binds and recruits the additional thermostable kinase(s) are present within the same hetero-multimeric fusion protein complex.

The additional thermostable kinase recruited to the antibody-analyte complexes (by the additional single domain antibody/ antibody fragment) may, for example, be any of the thermostable kinases described herein.

In one embodiment of the present invention, one or more alternative or additional "detector molecules" (as defined herein) may be used to detect the antibody-analyte complexes formed by the fusion protein complexes of the present invention (in addition to or instead of an ATP detection step) eg. as discussed in Example 14 part (ii).

By way of example, the detection step may employ one or more additional single domain antibodies or antibody fragments that bind and recruit the detector molecule(s) to the antibody-analyte complexes. The additional single domain antibody/ antibody fragment targeting the detector molecule may be present in the fusion protein targeting the analyte of interest (ie. the analyte being detected). Alternatively, the additional single domain antibody/ antibody fragment may be present in a different fusion protein from the fusion protein targeting the analyte(s) of interest. The fusion protein that binds and recruits the additional detector molecule can be present in the form of a single fusion protein or as part of a homo-multimeric or hetero-multimeric fusion protein complex.

Optionally, the fusion protein that targets the analyte(s) of interest and the fusion protein that binds and recruits the additional detector molecule(s) are present within the same hetero-multimeric fusion protein complex.

Examples of suitable detector molecules are described herein (eg. a fluorophore). The detector molecules described herein can be detected using any conventional means known in the art.

WO 00/46357 describes detection of analytes using an antibody conjugated to a 'reporter' thermostable kinase.

The fusion protein of the present invention may be used as a reporter molecule in a detection assay for identifying and/ or quantifying an analyte to which the single-chain antibody or antibody fragment binds.

Thus, in one aspect, the invention provides a reporter molecule for detecting an analyte on or within a sample, the reporter molecule comprising a fusion protein of the invention as defined in the claims.

The reporter molecule of the invention may comprise multiple (eg. 2, 3, 4, 5, 6, 7 or 8), different fusion proteins of the invention. In this regard, the fusion proteins may comprise different thermostable kinases and/ or different single-domain antibodies/ antibody fragments. By way of example, the different single-domain antibodies/ antibody fragments may bind different analytes, or may bind to the same or different epitopes on the same analyte.

In one embodiment, the receptor molecule of the invention comprises a multimeric fusion protein complex of the invention, as defined in the claims.

The invention also provides an *in vitro* method (as defined in the claims) of detecting an analyte on or within a sample, comprising:
(i) contacting the sample with a fusion protein or reporter molecule of the invention as defined in the claims, wherein the single-domain antibody or antibody fragment binds the analyte to form an antibody-analyte complex; and
(ii) detecting the antibody-analyte complex.

Detection of the antibody-analyte complex indicates that the analyte is present on or within the sample. If antibody-analyte complex is not detected, this indicates that the analyte is not present on or within the sample.

In accordance with this aspect of the invention, the fusion protein binds the analyte via the single-domain antibody or antibody fragment, to form an antibody-analyte complex. If antibody-analyte complexes are detected, this indicates that the fusion protein of the invention bound to analyte - ie. there was analyte present in the sample. If antibody-analyte complexes are not detected, this indicates that the fusion protein did not bind to analyte - ie. there was no analyte present in the sample.

One advantage of reporter molecules comprising fusion proteins or multimeric fusion protein complexes described herein that comprise multiple (eg. at least two) different antibody components binding different analytes is the ability to use a single reagent to simultaneously detect multiple different analytes.

The invention thus provides an *in vitro* method (as defined in the claims) of detecting multiple (eg. at least 2, 3, 4, 5, 6, 7 or 8) different analytes on or within a sample, comprising:
(i) contacting the sample with a fusion protein or reporter molecule comprising multiple (eg. at least two) antibody components, wherein said antibody components bind to said multiple different analytes to form antibody-analyte complexes, as defined in the claims; and
(ii) detecting the antibody-analyte complexes.

Detection of the antibody-analyte complexes indicates that the analytes are present on or within the sample.

Viruses (such as the influenza virus or norovirus) have the ability to rapidly mutate and undergo a shift in antigen diversity. Detection of virus infection is typically performed (in the art) using a single monoclonal antibody that targets a single virus antigen. Mutation of the antigen can prevent binding of the monoclonal antibody leading to a false negative signal. Polyclonal antibodies can be used to address this problem; however, they are slow to produce, and variable in quality.

In one embodiment, the present invention overcomes this problem by providing a single reagent (as defined in the claims) comprising multiple different antibody components that recognise multiple different antigens (eg. multiple different viral antigens). It is unlikely that all of the different target antigens will undergo mutation to evade detection by the antibody/ antibody fragment, and so the fusion proteins/ fusion protein complexes of the present invention advantageously reduce the risk of false negative results when determining (for example) the presence of a virus infection. An example of this aspect of the invention is described in Example 13 part (i).

The fusion proteins/ fusion protein complexes and reporter molecules described herein are also useful for detecting multiple different virus genotypes using a single reagent (where the different single domain antibody components of the fusion proteins/ fusion protein complexes bind to different virus genotypes). By way of example, a fusion protein/ fusion protein complex or reporter molecule of the invention as described herein are useful for simultaneously detecting the presence of the norovirus genotypes GI and GII (eg. as discussed in Example 13 part (i)).

The invention thus provides an *in vitro* method (as defined in the claims) of detecting multiple (eg. at least 2, 3, 4, 5, 6, 7 or 8) different viral genotypes (eg. norovirus genotypes GI and GII) on or within a sample, comprising:
(i) contacting the sample with a fusion protein or reporter molecule of the invention comprising multiple (eg. at least 2, 3, 4, 5, 6, 7 or 8) antibody components, wherein said antibody components bind to viral antigens from said multiple different genotypes to form antibody-antigen complexes, as defined in the claims; and
(ii) detecting the antibody-antigen complexes.

Detection of the antibody-antigen complexes indicates that the viral genotypes are present on or within the sample.

The ability to simultaneously detect multiple analytes using a single reagent is also an advantage when detecting the presence of bacterial infections - for example, for detecting the presence of multiple different bacterial infections, or for detecting the presence of a single bacterial infection associated with multiple different toxins that can mediate disease either individually or in combination. For example, *Clostridium difficile* can mediate disease through the expression of toxin B alone or in combination with toxin A. Hence, in one embodiment, a fusion protein/ fusion protein complex or reporter molecule of the invention as defined herein is useful to screen clinical isolates to detect the presence of *Clostridium difficile* expressing only toxin A or toxin B, or a combination of both toxin A and toxin B (eg. as discussed in Example 13 part (i)).

The invention thus provides an *in vitro* method (as defined in the claims) of detecting multiple (eg. at least 2, 3, 4, 5, 6, 7 or 8) different bacterial proteins (eg. *C. difficile* proteins such as toxin A and/ or toxin B) on or within a sample, comprising:
(i) contacting the sample with a fusion protein or reporter molecule comprising multiple (eg. at least 2, 3, 4, 5, 6, 7 or 8) antibody components, wherein said antibody components bind to said multiple different bacterial proteins to form antibody-protein complexes, as defined in the claims; and
(ii) detecting the antibody-protein complexes.

Detection of the antibody-protein complexes indicates that the bacterial proteins are present on or within the sample.

The term "sample" encompasses any item, instrument, surface, fluid or material. Examples include, but are not limited to, clinical samples (such as blood (eg. clarified whole blood), serum, sputum, oral samples such as saliva, pus, vaginal samples, stool samples, vomit); environmental samples (such water, soil, air samples); surgical and medical equipment and instruments such as endoscopes, microtitre plates, dipsticks, lateral flow devices, hospital gowns, bedclothes; bulk liquids; culled animal material; pharmaceuticals; workbenches, walls and floors. Reference to "contacting the sample" with a fusion protein embraces contacting a portion or an extract of the sample suspected of comprising the analyte.

Prior to carrying out the method of the invention, it is not necessarily known whether analyte is present on or within the sample. It is possible that the sample may not contain any analyte at all. In general, however, the analyte is known to be present on or within the sample. In one embodiment, the aim of the assay is to identify whether or not analyte is present on or within the sample. In one embodiment, the aim of the assay is to quantify analyte known to be present on or within the sample.

In one embodiment, the method of the invention comprises the step of treating the sample in order to (substantially) remove any endogenous kinase present on or within the sample. This treatment step may be conducted prior to, after, or simultaneously with the step of contacting the sample with the fusion protein or reporter molecule of the invention, so long as it is conducted prior to the step of detecting the antibody-analyte complex by detecting the kinase activity of the thermostable kinase. In one embodiment, the removal of endogenous kinase is performed prior to contacting the sample with the fusion protein or reporter molecule of the invention. This treatment step ensures that the signal obtained from the assay is not contaminated or otherwise adversely affected by any endogenous kinase present in the sample being tested (ie. reduces the risk of "false positive" readings).

Reference to "removing kinase" from the sample embraces removing kinase activity, such as by denaturing or otherwise inactivating any endogenous kinase *in situ.* By way of example, removal of endogenous mesophilic kinase can conveniently be achieved by heating the sample to a temperature at which any endogenous mesophilic kinase (if present) is denatured or rendered incapable of catalysing the formation of ATP, but at which the thermostable kinase present in the fusion protein of the invention is not denatured and retains its kinase activity. A suitable treatment might be to heat the sample to 60 to 90°C for at least 10 minutes (such as 70°C for at least 30 minutes, or at 80°C for at least 10 minutes). Alternatively, other treatments might be appropriate to destroy the activity of endogenous kinase, whilst retaining activity of the thermostable kinase of the invention, such as the use of ultrasound, or extremes of pH or salt concentration.

In one embodiment, after the step of contacting the sample with the fusion protein or reporter molecule of the invention, and before the step of detecting the antibody-analyte complex, the method of the invention comprises removing any fusion protein that is not bound to analyte. This step ensures that the signal obtained from the assay is not contaminated or otherwise adversely affected by kinase added to the sample that does not bind the analyte of interest (ie. reduces the risk of "false positive" readings).

In one embodiment, antibody-analyte complexes may be detected by detecting the kinase activity of the thermostable kinase component of the fusion protein.

The kinase activity of the thermostable kinase can be measured by any conventional means known in the art. In one embodiment, the thermostable kinase component of the fusion protein provides a highly specific output in the form of ATP generation and hence light generation, which allows for highly sensitive and specific detection of the binding event between the antibody/ antibody fragment and the analyte. The detection limits are at or below the dissociation constant (Kd) of the antibody, providing a highly sensitive assay.

In one embodiment, the kinase activity of the bound thermostable kinase in the antibody-analyte complex is detected by adding a substrate (eg. ADP), and detecting the formation of ATP. The ATP may be detected and measured directly, but is more usually detected indirectly, eg. by reaction of the ATP with a bioluminescent reagent such as luciferin/luciferase, to generate light. The light output from the assay (ie. the light emitted by the reaction of ATP with the bioluminescent reagent) can be measured using conventional techniques known in the art, such as using a luminometer.

Detection of ATP indicates that the assay contained the fusion protein of the invention bound to analyte - ie. analyte was present in the sample. If no ATP is detected, this indicates that the fusion protein did not bind to analyte - ie. analyte was not present in the sample.

In one embodiment, prior to addition of ADP and detection of kinase activity, the method comprises the step of removing any endogenous ATP present on or within the sample. This step further decreases the background noise in the assay. The removal of endogenous ATP may be achieved by any conventional method known in the art - for example, by adding an ATPase such as a thermolabile ATPase that can itself be destroyed by use of elevated temperature (to avoid the ATPase adversely influencing the signal obtained from the thermostable kinase).

In one embodiment, the method comprises the following steps:
1. A sample containing or suspected of containing an analyte of interest is contacted with a fusion protein or reporter molecule of the invention comprising a single-domain antibody or antibody fragment that binds the analyte of interest;
2. The fusion protein binds to analyte in the sample (if present) via the single-domain antibody or antibody fragment, to form antibody-analyte complexes;
3. The assay is treated (eg. by washing) to remove unbound components of the sample and unbound fusion protein, but so as to retain any antibody-analyte complexes;
4. Optionally, a thermolabile ATPase is added to the solid phase to remove any endogenous ATP;
5. The assay is treated (eg. by heating) to destroy the thermolabile ATPase (if added) and to denature any endogenous kinase that may be present, but so as not to denature the thermostable kinase in the fusion protein;
6. ADP is added to the assay, as a substrate for the thermostable kinase; and the assay is tested for presence and/ or amount of ATP (eg. by adding a bioluminescent compound and detecting/ measuring the light output).

In one embodiment, the ATP detection signal generated by the antibody-analyte complexes is further amplified using one or more additional single domain antibodies or antibody fragments that bind and recruit one or more additional thermostable kinase(s) to the antibody-analyte complexes.

In one embodiment, the additional single domain antibody/ antibody fragment may be present in the fusion protein targeting the analyte of interest (ie. the analyte being detected). Alternatively, the additional single domain antibody/ antibody fragment may be present in a different fusion protein from the fusion protein targeting the analyte(s) of interest. The fusion protein comprising the single domain antibody/ antibody fragment that binds and recruits an additional thermostable kinase can be present in the form of a single fusion protein, or as part of a homo-multimeric and hetero-multimeric fusion protein complex.

Optionally, the fusion protein that targets the analyte(s) of interest and the fusion protein that binds and recruits the additional thermostable kinase(s) are present within the same hetero-multimeric fusion protein complex.

In an alternative embodiment of the present invention, one or more alternative or additional "detector molecules" may be used to detect the antibody-analyte complexes formed by the fusion protein complexes of the present invention (in addition to or instead of an ATP detection step) eg. as discussed in Example 14 part (ii).

By way of example, the detection step may employ one or more additional single domain antibodies or antibody fragments that bind and recruit the detector molecule(s) to the antibody-analyte complexes. The additional single domain antibody/ antibody fragment targeting the detector molecule may be present in the fusion protein targeting the analyte of interest (ie. the analyte being detected). Alternatively, the additional single domain antibody/ antibody fragment may be present in a different fusion protein from the fusion protein targeting the analyte(s) of interest. The fusion protein comprising the single domain antibody/ antibody fragment that binds and recruits an additional detector molecule can be present in the form of a single fusion protein or as part of a homo-multimeric or hetero-multimeric fusion protein complex.

Optionally, the fusion protein that targets the analyte(s) of interest and the fusion protein that binds and recruits the additional detector molecule(s) are present within the same hetero-multimeric fusion protein complex.

Examples of suitable detector molecules are described herein (eg. a fluorophore). The detector molecules described herein can be detected using any conventional means known in the art.

In one embodiment, detection of the detector molecule is performed in addition to detecting the kinase activity of the thermostable kinase component of the fusion protein (and/or the kinase activity of any additional thermostable kinase recruited to the antibody-analyte complex). Alternatively, detection of the detector molecule may be performed instead of detecting the kinase activity of the thermostable kinase component of the fusion protein (and/or the kinase activity of any additional thermostable kinase recruited to the antibody-analyte complex).

In one embodiment described herein, the additional reporter or tracer molecules recruited to the antibody-analyte complexes may be used to detect the presence of the analyte(s) of interest in a whole body system or in isolated tissue samples by histology.

The term "analyte" encompasses both infectious and non-infectious agents derived from a biological source. Examples of analytes include bacteria, viruses, fungi, prions, toxins, allergens, spores, and fragments and derivatives/ components of any of the foregoing. In one embodiment, an analyte may be referred to as a 'contaminant' or a 'contaminating biological agent'.

The present invention is applicable to detection of any analyte against which a single-domain antibody can be raised. In other words, any analyte can be detected using the present invention, so long as it can be bound by the fusion protein of the invention via the single-domain antibody or antibody fragment.

In one embodiment, the analyte comprises or consists of one or more proteins, nucleic acids, carbohydrates and/ or lipids. In one embodiment, the analyte comprises or consists of a virus, or a cell such as a bacterium or yeast.

In one embodiment, the analyte is derived from a pathogen, such as a pathogenic bacterium or virus.

In one embodiment, the analyte comprises or consists of a protein, such as a protein selected from the group consisting of an animal (eg. mammal such as human) protein, a bacterial protein, a viral protein, a plant protein or a fungal protein. In one embodiment, the analyte comprises or consists of a blood protein or a protein derived from neurological tissue.

In one embodiment, the analyte comprises or consists of a bacterial protein from a bacterial species that may be a pathogen of humans, animals or plants and may be found in clinical samples, environmental samples, or process samples. The bacterial protein may, for example, be selected from the group consisting of a bacterial fimbrial protein (eg. CgsA from *E*. *coli* and AgfA from *Salmonella*), a bacterial toxin protein (eg. toxins from *Bacillus* species, such as *Bacillus anthracis, Corynebacterium diphtheriae, Clostridial* species, such as *Clostridium botulium* (eg. *C. botulinum* neurotoxin (BoNT)) and *Clostridium difficile* (eg. *C*. *difficile* toxin A and toxin B), *Staphylococcus* species, such as *Staphylococcus aureus* (eg. *S aureus enterotoxin B)*), a bacterial enzyme, such as a bacterial metalloprotease (eg. New Delhi metalloprotease produced by *E*. *coli and Klebsiella*); a bacterial cell surface protein (eg. peptidoglycan, lipoproteins); and a bacterial spore protein (eg. from Gram positive bacteria).

In one another embodiment, the analyte comprises or consists of a viral protein from a virus that may be a pathogen of humans, animals, bacteria or plants and may be found in clinical samples, environmental samples, or process samples. The viral protein may be a nucleoprotein, a viral envelope protein, a viral capsid protein, or a viral core protein. Examples of viral proteins are from a bacteriophage virus (eg. the MS2 and PP7 proteins), norwalk virus (eg. capsid protein), rotavirus (eg. VP2, VP6 and VP7 proteins), coronavirus (eg. SARS S, E and M proteins), bluetongue virus (eg. VP2 protein), human papillomavirus (eg. viral major structural protein, L1), hepatitis B (eg. small envelope protein HBsAg), Hepatitis C virus (eg. core, E1 and E2 proteins), influenza virus (eg. neuraminidase and haemagglutinin and matrix proteins), poliovirus (eg. capsid VP0, 1 and 3 proteins), HIV (eg. Pr55gag, envelope proteins), dengue B virus (eg. envelope (e) and pre-membrane / membrane (prM/M), ebola virus and norovirus.

In another embodiment, the analyte comprises or consists of a fungal protein selected from the group consisting of cell wall proteins (eg. SC3 from *Schizophyllum commune*, RodA/B from *Aspergillus fumigates,* and equivalent proteins from yeast), fungal spore proteins, hyphal proteins, mycotoxins, and fungal prions (eg. Sup35, Het S, URE 2, Rnq1, New 1).

In one embodiment, the analyte comprises or consists of a blood protein selected from the group consisting of blood clotting proteins (eg. fibrinogen, fibrin peptides, fibrin, transglutaminase substrates, thrombin), serum proteins (e.g. albumin and globulin), platelet proteins, blood cell glycoproteins, factor VIII, and haemoglobin.

In yet another embodiment, the analyte comprises or consists of a self-aggregating protein selected from the group consisting of prions (eg. PrP^{Sc} and PrP^{c}, Sup35, Het S, Ure 2, Rnq1, New 1), prion mimetic proteins, amyloid fibrils, (eg. amyloid beta), and misfolded proteins (eg. alpha-synuclein).

In one embodiment, the analyte comprises or consists of a small molecule that is a substance of abuse. Examples of such molecules include nicotine and cocaine.

In another embodiment, the analyte comprises or consists of a nucleic acid molecule, such as a DNA molecule or an RNA molecule. By way of example, the nucleic acid analyte may comprise or consist of single-stranded DNA (ssDNA), single-stranded RNA (ssRNA), double-stranded DNA (dsDNA) or double-stranded RNA (dsRNA). In one embodiment, the nucleic acid molecule comprises or consists of a nucleic acid sequence that encodes any of the protein analytes defined above.

In another embodiment, the analyte comprises or consists of a carbohydrate, such as a carbohydrate selected from the group consisting of exopolysaccharide, lipopolysaccharide (EPS/LPS, sometimes known as endotoxin) (eg. from *Legionella* species, *E*. *coli, Staphylococcus* species, *Streptococcus* species, *Pseudomonas* species, *Acinetobactor* species, *Campylobactor* species, and *Bacillus* species), peptidoglycan, cell wall components of plants, fungi and yeast (eg. chitin, lignin, glucan), mucin preparations, glycolipids (eg. brain derived glycolipids), glycoproteins (eg. cell surface glycoproteins, Eap1 p), spore extracts (eg. from *Bacillus spp, Clostridial spp* and other spore-formers), polysaccharides from yeast capsules, and invertebrate secretions (eg. from molluscan gels).

In another embodiment, the analyte comprises or consists of a lipid, such as a lipid selected from the group consisting of glycolipids (eg. brain-derived glycolipids), gangliosides (eg. neuronal cell gangliosides such as GT_{1b}, GT₁ₐ and gangliosides of more general cell origin such as GM₁), and plant oils and lipids.

In another embodiment, the analyte may be derived from or may comprise/ consist of one or more components of blood, serum, albumin, mucus, egg, neurological tissue, food, culled animal material, or one or more components present in soil, water or air samples. In yet another embodiment of the invention, the biological matrix comprises one or more components selected from the group consisting of fibrinogen, thrombin, factor VIII, CaCl₂, and, optionally, albumin and/ or haemoglobin.

In another embodiment, the term "analyte" also encompasses molecules having a detector function, which are provided as part of the detection reaction, as discussed above. In the present invention, analytes having a detector function ("detector molecules") may be provided to increase the sensitivity of the detection reaction for detection of biological analyte(s) of interest (eg. as discussed above).

In one embodiment, a detector molecule may comprise or consist of a thermostable kinase (such as an adenylate kinase, acetate kinase, UMP kinase, pyruvate kinase or butyrate kinase or any thermostable kinase described herein).

In another embodiment, the detector molecule may comprise or consist of a fluorophore, hapten (eg. biotin or digoxigenin), phosphatase, peroxidase, acridine ester, radioisotope (eg. 32P, 35S) or any other conventional reporter molecule known in the art. In another embodiment, the detector molecule may comprise or consist of a tracer, such as an isotopic tracer (eg. ²H, ¹³C , and ¹⁵N), or any other conventional tracer molecule known in the art.

The fusion proteins may be used in a variety of assay formats familiar to those with knowledge of the art. Accordingly, in one embodiment, the fusion protein or a reporter molecule of the invention is attached to a solid support. Suitable examples of assay formats include an enzyme-linked immunoassay (ELISA) or variant thereof, a magnetic bead-based solid support assay, a Western Blot, an autoanalyser, a nucleic acid amplification system, a lateral flow or dipstick assay or other rapid assay format.

In a further aspect of the invention, there is provided a detection kit comprising: (i) one or more fusion proteins, fusion protein complexes or reporter molecules of the invention as defined in the claims, and (ii) a substrate for the multimeric thermostable kinase.

In one embodiment, the kit comprises a multimeric fusion protein complex comprising multiple fusion proteins of the invention (as defined in the claims).

In one embodiment, the substrate for the thermostable kinase is ADP.

In one embodiment, to measure the thermostable kinase amount or activity, the kit may also include means for detecting ATP, such as a bioluminescent reagent (e.g. luciferin/luciferase) and optionally a luminometer.

From previous testing with known analytes, data can be prepared correlating the amount of kinase activity with the amount or activity of the analyte. The kit may therefore further comprise a look-up table correlating the observed kinase activity with the amount or activity of a list of specified analytes.

In one embodiment, the kit may further comprise one or more detector molecules (as defined herein) that can be used to detect antibody-analyte complexes in addition to (or instead of) detecting the thermostable kinase activity of the fusion protein. In one embodiment, the additional reporter molecule may comprise or consist of one or more additional thermostable kinases, fluorophores, biotin, digoxigenin, phosphatase, peroxidase, and other conventional reporter or tracer molecules known in the art. The kit may also comprise conventional means for detecting the detector molecule(s).

The invention is now described in specific embodiments in the following examples and with reference to the accompanying drawings.

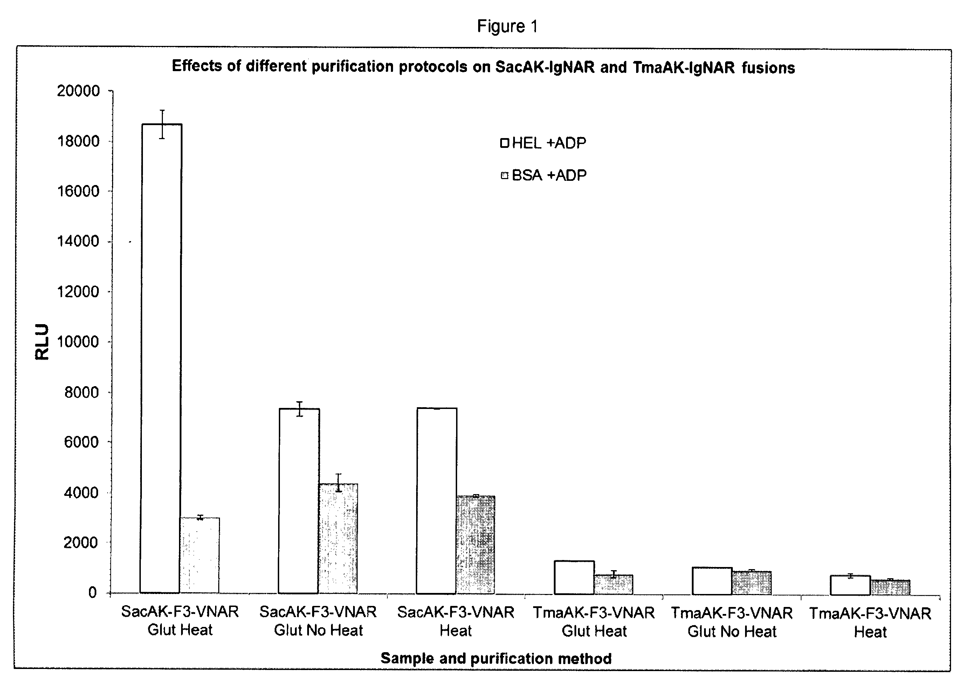

**Figure 1** illustrates functional recovery of correctly folded single-domain antibody (V_{NAR}) in the presence of the Sac tAK domain and glutathione. The fusion proteins were assessed for their ability to bind to hen egg lysozyme immobilized onto a microtitre plate in an ELISA format, using the tAK activity as the assay read-out. In each case background binding was measured using BSA as a non-relevant antigen control. In the case of Tma tAK fusions, no significant increase in binding was observed above the background binding to BSA with either glutathione extraction, heat treatment or a combination of the two. In contrast, for the Sac tAK fusions, heat or extraction in the presence of glutathione resulted in an increase in productive binding above the BSA control, whilst the combination of the two factors gave approximately 10-fold increase in binding.

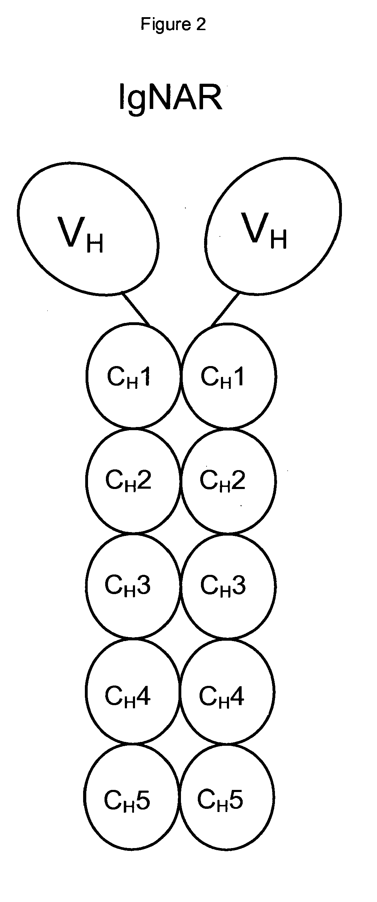

**Figure 2** provides a diagrammatic representation of shark Ig new antigen receptors (V_{NAR})·
**Figure 3** illustrates construction of a SacAK-F3-V_{NAR} fusion or SacAK-F3-V_{H} fusion. SacAK was cloned into a Gateway-adapted pMTL1015 vector (Ungurs *et al.* 2010), upstream of the recombination site. The V_{NAR} or V_{H} was recombined from an entry vector to generate the first generation construct. Where required, additional linker regions (eg. (GGGGS)₃) were cloned to replace the intergene region using engineered restriction enzyme sites as shown.

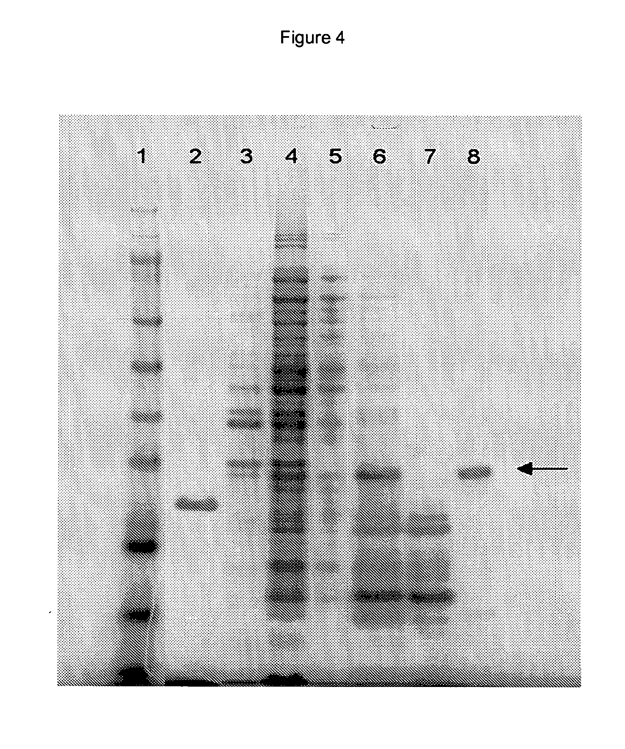

**Figure 4** illustrates SDS page analysis of purification of SacAK-F3-V_{NAR} fusion. Equal amounts of protein from each stage of the purification was loaded onto a 4-12% Bis Tris gel and electrophoresed in MES buffer under standard conditions. Purified SacAK-F3- VNAR is indicated with an arrow and lanes loaded as follows: 1: See Blue plus 2 ladder, 2: SacAK control (2.5µg), 3: Insoluble homogenate, 4: Soluble homogenate, 5: Insoluble thermoprecipitate, 6: Soluble thermoprecipitate/column load, 7: Blue Sepharose column wash, 8: Blue Sepharose column eluate

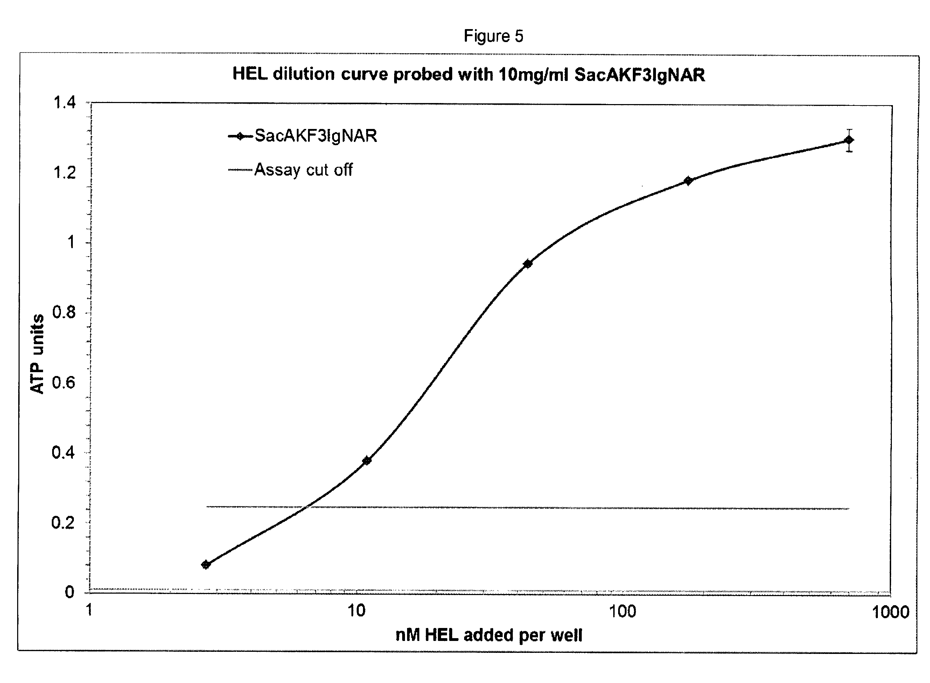

**Figure 5** illustrates detection of HEL by SacAK-F3-V_{NAR} at close to the estimated KD of the V_{NAR} at around 20nM (Dooley *et al*.). Dilutions of HEL with final concentrations from 5-500nM were generated in PBS, 100ul was coated onto white Maxisorp plate and blocked with 5% BSA. The plate was probed with 10µg/ml SacAK-F3-VNAR and the signal (corrected for background) was plotted. The level of sensitivity was defined as 3 SD above the blank (no antigen) control value. Results are typical of more than one experiment (n=6).

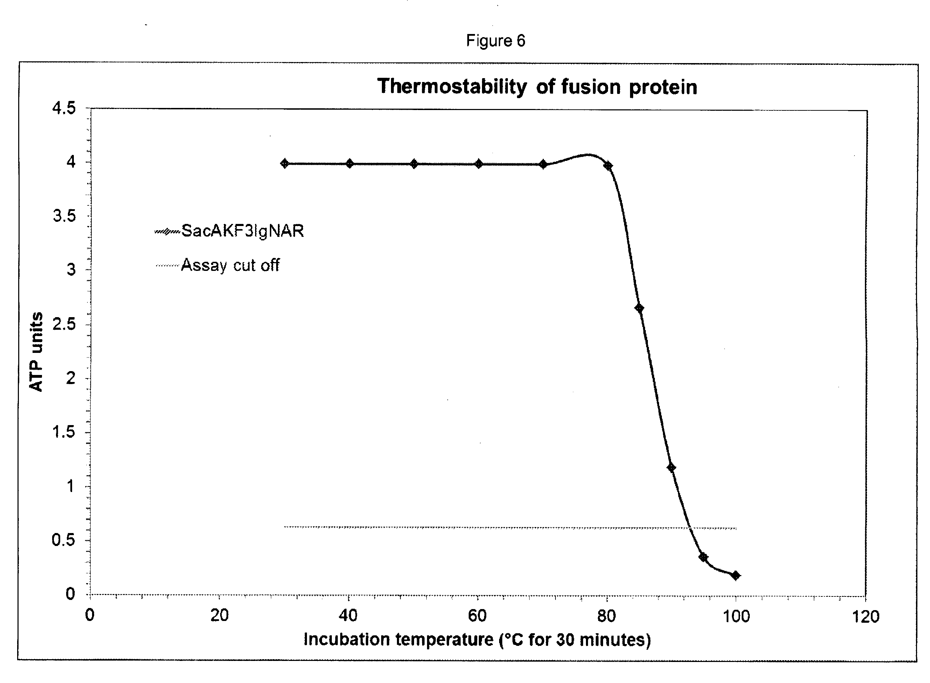

**Figure 6** illustrates retention of SacAK-F3-V_{NAR} binding and enzymatic activity after heat treatment at above 80°C. 10µg/ml aliquots of SacAK-F3-V_{NAR} were incubated at a range of temperatures, cooled to room temperature then used to probe a plate coated with 10µg/ml HEL. Activity of the fusion was unaffected up to 80°C, between 80-100°C the signal dropped off significantly, with the melting temperature (temperature at which fusion retains half maximal activity) estimated at around 88.9°C. SacAK alone at equimolar concentrations and under the same salt concentrations, showed similar/different melting curves.
   Similar results were observed with the Sac-F3-V_{H} fusion protein (results not shown) with activity of the Sac domain unaffected by temperatures up to 80°C and approximately 10% of activity retained after 30 minutes treatment at 90°C.

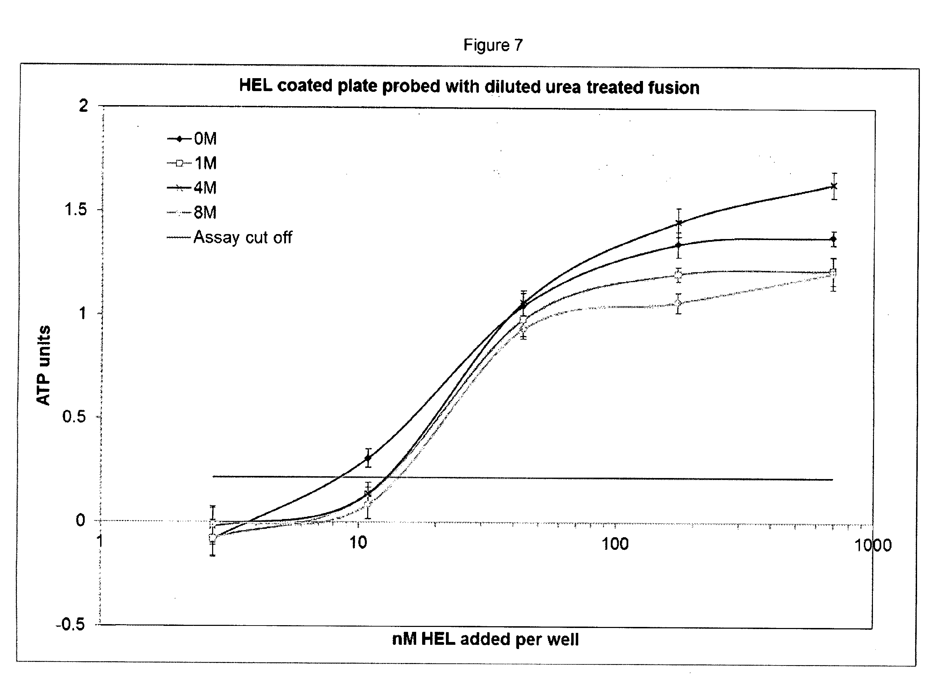

**Figure 7** illustrates that SacAK-F3-VNAR fusions retain activity after treatment with urea. 100µg/ml of the fusion protein was treated with a final concentration of 0, 1, 4 or 8M urea for 30 minutes at 37°C. The sample was immediately diluted 1:10 and used to probe a Maxisorp plate coated with dilutions of HEL. The limits of detection of the assay, as defined by 3SD above the no antigen control value, remained unchanged under the conditions tested. Treatment of the bound HEL with the same concentration of urea reduced but did not abolish binding by the fusion (results not shown). Results are typical of more than one experiment (n=4).

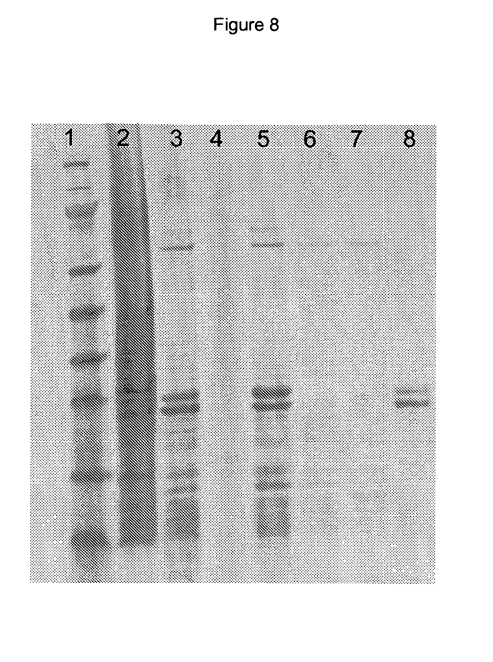

**Figure 8** illustrates SDS page analysis of purification of SacAK-F3-V_{H} fusion. Equal amounts of protein from each stage of the purification was loaded onto a 4-12% Bis Tris gel and electrophoresed in MES buffer under standard conditions. Purified SacAK-F3-VH is indicated with an arrow and lanes loaded as follows: 1: See Blue plus 2 ladder, 2: Insoluble homogenate, 3: Soluble homogenate, 4: Insoluble thermoprecipitate, 5: Soluble thermoprecipitate/column load, 6 Blue Sepharose column flow through 7: Blue Sepharose column wash, 8: Blue Sepharose column eluate. The Sac-F3-VH fusion runs consistently as a double band of approximate molecular weight 28kDa consistent with observed migration of Sac fusion proteins on SDS-PAGE.

### LIST OF SEQ ID NOs

**SEQ ID NO 1: Acetate kinase from *Methanosarcina thermophila***
   1 mkvlvinags sslkyqlidm tnesalavgl cerigidnsi itqkkfdgkk lekltdlpth
   61 kdaleevvka Itddefgvik dmgeinavgh rvvhggekft tsalydegve kaikdcfela
   121 plhnppnmmg isacaeimpg tpmvivfdta fhqtmppyay myalpydlye khgvrkygfh
   181 gtshkyvaer aalmlgkpae etkiitchlg ngssitaveg gksvetsmgf tpleglamgt
   241 rcgsidpaiv pflmekeglt treidtlmnk ksgvlgvsgl sndfrdldea askgnrkael
   301 aleifaykvk kfigeysavl ngadavvfta gigensasir kriltgldgi gikiddeknk
   361 irgqeidist pdakvrvfvi ptneelaiar etkeivetev klrssipv
**SEQ ID NO 2: Pyruvate kinase from *Geobacillus stearothermophilus***
   1 mkrktkivct igpasesvdk Ivqlmeagmn varlnfshgd heehgrrian ireaakrtgr
   61 tvailldtkg peirthnmen gaielkegsk Ivismsevlg tpekisvtyp sliddvsvga
   121 killddglis levnavdkqa geivttvlng gvlknkkgvn vpgvkvnlpg itekdradil
   181 fgirqgidfi aasfvrrasd vleirellea hdalhiqiia kieneegvan ideileaadg
   241 Imvargdlgv eipaeevpli qkllikkcnm Igkpvitatq mldsmqrnpr ptraeasdva
   301 naifdgtdav mlsgetaagq ypveavktmh qialrteqal ehrdilsqrt kesqttitda
   361 igqsvahtal nldvaaivtp tvsgktpqmv akyrpkapii avtsneavsr rlalvwgvyt
   421 keaphvnttd emldvavdaa vrsglvkhgd Ivvitagvpv getgstnlmk vhvisdllak
   481 gqgigrksaf gkavvaktae earqkmvdgg ilvtvstdad mmpaiekaaa iiteegglts
   541 haavvglslg ipvivgvena ttlfkdgqei tvdggfgavy rghasvl
**SEQ ID NO 3: UMP kinase, eg. from *Pyrococcus furiosus***
   1 gsshhhhhhs sglvprgshm rivfdiggsv Ivpenpdidf ikeiayqltk vsedhevaw
   61 vgggklarky ievaekfnss etfkdfigiq itranamlli aalrekaypv wedfweawk
   121 avqlkkipvm ggthpghttd avaallaefl kadllvvitn vdgvytadpk kdptakkikk
   181 mkpeelleiv gkgiekagss svidplaaki iarsgiktiv igkedakdlf rvikgdhngt
   241 tiep
**SEQ ID NO 4: Butyrate kinase from *Thermotoga maritima***
   1 mfriltinpg ststklsife dermvkmqnf shspdelgrf qkildqlefr ekiarqfvee
   61 tgyslssfsa fvsrgglldp ipggvylvdg Imiktlksgk ngehasnlga iiahrfsset
   121 gvpayvvdpv vvdemedvar vsghpnyqrk sifhalnqkt vakevarmmn kryeemnlvv
   181 ahmgggisia ahrkgrvidv nnaldgdgpf tpersgtlpl tqlvdlcfsg kftyeemkkr
   241 ivgngglvay Igtsdarevv rrikqgdewa krvyramayq iakwigkmaa vlkgevdfiv
   301 Itgglaheke flvpwitkrv sfiapvlvfp gsneekalal salrvlrgee kpknyseesr
   361 rwrerydsyl dgilr
**SEQ ID NO 5: Adenylate kinase (monomeric) from *Thermotoga maritima***
   1 mmaylvflgp pgagkgtyak rlqeitgiph istgdifrdi vkkendelgk kikeimerge
   61 Ivpdelvnev vkrrlsekdc ergfildgyp rtvaqaefld gflktqnkel taavlfevpe
   121 ewvqrltar ricpkcgriy nlislppked elcddckvkl vqreddkeet vrhrykvyle
   181 ktqpvidyyd kkgilkrvdg tigidnviae vlkiigwsdk
**SEQ ID NO 6: Adenylate kinase (trimeric) from *Sulfolobus acidocaldarius***
   1 mkigivtgip gvgkstvlak vkeildnqgi nnkiinygdf mlatalklgy akdrdemrkl
   61 svekqkklqi daakgiaeea raggegylfi dthavirtps gylpglpsyv iteinpsvif
   121 Ileadpkiil srqkrdttrn rndysdesvi letinfarya atasavlags tvkvivnveg
   181 dpsiaaneii rsmk
**SEQ ID NO 7: Gene sequence for adenylate kinase from *Sulfolobus acidocaldarius* with unstructured linker and V_{NAR} sequence**
**SEQ ID NO 8: V_{NAR} sequence for hen egg lysozyme specific single domain antibody from shark.**
**SEQ ID NO 9: Fusion protein sequence for adenylate kinase from *Sulfolobus acidocaldarius* with unstructured linker and V_{NAR}** sequence
**SEQ ID NO 10: Fusion protein sequence for adenylate kinase from *Sulfolobus acidocaldarius* with helical linker and V_{NAR} sequence**
**SEQ ID NO 11: Fusion protein sequence for V_{NAR} sequence with helical linker and adenylate kinase from *Sulfolobus acidocaldarius***
**SEQ ID NO 12: Fusion protein sequence for V_{NAR} sequence with unstructured linker and adenylate kinase from *Sulfolobus acidocaldarius***
**SEQ ID NO 13: V_{H}H sequence for hen egg lysozyme specific single domain antibody from camelid**
**SEQ ID NO 14: Fusion protein sequence for adenylate kinase from** *Sulfolobus **acidocaldarius*** with **unstructured linker and V_{H}H sequence**
**SEQ ID NO 15: Fusion protein sequence for adenylate kinase from *Sulfolobus acidocaldarius* with helical linker and V_{H}H sequence**
**SEQ ID NO 16: Fusion protein sequence for V_{H}H sequence with helical linker and adenylate** kinase **from *Sulfolobus acidocaldarius***
**SEQ ID NO 17: Fusion protein sequence for V_{H}H sequence with** unstructured **linker and adenylate kinase from *Sulfolobus acidocaldarius***
**SEQ ID NO: 18: Helical linker**
   LAEAAAKEAAAKEAAAKEAAAKAAA
**SEQ ID NO: 19: Acetate kinase from *Thermotoga maritima***
**SEQ ID NO: 20: UMP kinase from *Thermotoga maritima.***
**SEQ ID NO: 21: UMP kinase from *Sulfolobus acidocaldarius.***

### EXAMPLES

### Example 1: Cloning of IgNAR gene constructs

*Sulfolobus acidocaldarius* adenylate kinase (SacAK: GenBank: YP255258) coding sequence was synthesised using a high expressing *E. coli codon* bias and ligated into a pMTL1015 vector (GenBank: CS249842) using Ndel (R0111 S) and Sall-HF (R3138S). A Gateway™ cassette (11828029, Invitrogen, Paisley, UK) was subsequently ligated into the Xhol (R0146S) site. The Xhol site had previously been blunted using T4 DNA polymerase (M0203S) prior to the ligation. The resulting cytoplasmic expression destination vector was called pTDCnSacAK-DEST. The destination vector was cloned into ccdB survival chemically competent cells (11828029, Invitrogen, Paisley, UK).

The HEL specific V_{NAR} ([E] GenBank: AAN75851) was synthesised with a high expressing *E.coli* codon bias and a BamHI site at the 5' end then inserted into the commercially available pENTR/D-TOPO (Invitrogen). Cloning for the entry vector was carried out using chemically competent TOP10 cells (C404006, Invitrogen, Paisley, UK).

Recombination of the destination vector and entry vector was carried out using LR clonase II mix (11791020, Invitrogen, Paisley, UK) as per the manufacturer's instructions.

The gateway linker region, consisting of the gateway recombination site, was replaced with a flexible linker (F3) with the amino acid repeats [GGGGS]₃. The linker region was created as oligonucleotides (Sigma-Genosys, UK) with overlapping ends corresponding to the cleaved BamHI and SalI restriction sites. The oligonucleotides were annealed by incubating at 90°C prior to insertion through traditional restriction digest of the vector and ligation.

Completed expression vectors were then transformed into *E*. *coli* strain RV308 and stored in cryoprotective beadstocks (PL170, Microbank, Prolab Diagnostics, Canada).

Synthesis and recombination of the constructs was confirmed by sequencing (Beckman Coulter Genomics, Takeley, UK) as was the insertion of alternative linker regions in the fusion protein.

Single domain antibody and/or single domain antibody fragment fusions may alternatively be rapidly generated due to the cassette-based method of construction used in assembling the fusion constructs (Figure 1). Genetically engineered variants of SacAK or different thermostable kinases (e.g T.maritima acetate kinase or UMP kinase) can be inserted as NdeI-SalI fragments into the fusion construct to generate, for example, acetate kinase-F3 linker-single domain antibody fusions. Similarly, individual single domain antibody fragments or libraries of such fragments could be cloned into the fusions constructs as BamHI-Xhol fragments. This cassette approach circumvents the need for multiple cloning steps or any difficulties encountered with the recombination-based cloning systems (Gateway).

### Example 2: Expression of IgNAR fusion proteins in E. coli

Expression was achieved using *E*. *coli* RV308 strain in Modified Terrific Broth (T0918). Primary seed (Luria-Bertani Broth + Tetracycline (T7660) 10 µg/ml) was inoculated with one colony from a Luria-Bertani Agar + Tetracyline (10 µg/ml) plate and left to grow overnight at 200rpm and 37°C.

After overnight growth the primary seed was inoculated at 5ml/litre into the Modified Terrific Broth flask culture (+ 10 µg/ml Tet); 200rpm 37°C with OD₆₀₀ monitored. When an OD₆₀₀>4 was achieved the cells were harvested by centrifugation at 3000rpm for 30 minutes using a RC3BP (Sorvall, UK) centrifuge with H6000A rotor at 4°C.

The resulting cell paste was then resuspended in PBS and stored as aliquots at - 80°C until needed.

### Example 3: Purification of fusion protein

Cell paste was homogenised to break the cells in a high salt buffer (20mM Trizma-Base (T6066), 0.9M NaCl (S9888), 10mM MgCl (H8266) pH8.0) with 2mM Glutathione (G4251) added. The soluble fraction of the homogenate was then incubated at 80°C for 20 minutes to precipitate out all non-thermostable proteins. Following centrifugation the soluble fraction was loaded onto a 5ml Blue Sepharose FF Hitrap column (GE Healthcare, 17-0413-01) which was equilibrated in the same high salt buffer that was used to resuspend the cell paste for homogenisation. The glutathione was removed using a 1 hour gradient into 20mM Trizma-base, 0.9M NaCl, 10mM MgCL pH8.0. The fusion protein was then eluted using 20mM Trizma-base, 0.9M NaCl, 10mM MgCL, 10mM ATP (A3377) and AMP (A1752) pH8.0. The column was run at 1ml minute⁻¹, fractions were collected and analysed by SDS-PAGE gel and the fractions containing the final fusion protein product were pooled.

SDS-PAGE gels were run on NuPage 4-12% BisTris 15 well gels (NP0323BOX, Invitrogen, Paisley, UK) with NuPage MES SDS Running Buffer (20x) (NP0002, Invitrogen, Paisley, UK) with See Blue plus 2 ladder (LC5925, Invitrogen, Paisley, UK), 2.5 µg recombinant SacAK as on-gel controls. All samples were run in the presence of 5 µM DTT(D0632). Gels were developed using Simply Blue Safe Stain (LC6065, Invitrogen, Paisley, UK) and imaged using the GeIDoc.

### Example 4: Characterisation of fusion protein activity

White F96 MicroWell™ MaxiSorp plates (436110, Nunc, Denmark) were used in all assays.

All assays were read at the same gain setting (2553) on the BMG Labtech FLUOstar Omega plate reader.

In order to eliminate plate to plate variability each plate was also set up with an ATP dilution series from 5 down to 0.625 µMol per litre and this was used to convert the RLU to ATP units which are not subject to plate to plate variability.

### 4.1: Determination of AK activity

Equimolar SacAK-F3-V_{NAR} fusion protein (50ul) or the SacAK (50 µl) was added per well with 50 µl Luciferase reagent (Biothema ATP kit SL 144-04, Biothema AB, Sweden) + 15 µM ADP (Sigma A1752, ATP depleted using 5ml DEAE Sepharose FF HiTrap column (GE Healthcare, 17-5055-01)). The plate was incubated for 1 minute and luminescence read on a BMG Labtech FLUOstar Omega at 2553 gain as an endpoint reading after 15 seconds integration.

### 4.1: Detection of antigen-binding activity of V_{NAR}

Functional ELISA was tested by coating the plate with a serial dilution of HEL (L6876) made up in PBS from 1 µg/well down with an additional BSA (A6003) control strip (50µg/ml), for 1 hour shaken at 37°C. The plate was washed 3 times in PBS containing 1% Tween 20 (P1379) and then blocked in 5% BSA in PBS for 1 hour shaken at 37°C. The plate was washed 3 times in PBS containing 1% Tween 20. The fusion protein was bound to the plate at 10 µg/ml on both the HEL and BSA coated sides for 1 hour shaken at 37°C and then washed in PBS containing Tween. Luciferase reagent (Biothema ATP kit SL 144-04, Biothema AB, Sweden) with the addition of 15µM ADP (Sigma A1752, ATP depleted using 5ml DEAE Sepharose FF HiTrap column (GE Healthcare, 17-5055-01)) was added to the plate. The plate was incubated for 1 minute and luminescence read as Relative Luminescence Units (RLU) on a BMG Labtech FLUOstar Omega at 2553 gain as an endpoint reading after 15 seconds integration.

### Example 5: Demonstration of stability

### 5.1 Thermostability studies

A plate was coated with 100ul/well of 10µg/ml HEL (L6876) as well as a BSA (A6003) control for 1 hour shaken at 37°C. The plate was washed 3x in PBS + Tween 20 (P1379) and then blocked in 5% BSA in PBS for 1 hour shaken at 37°C. The plate was washed 3x in PBS + Tween 20.

Purified, characterised fusion (10µg/ml) was incubated in separate tubes for 30 minutes at a range of temperatures up to 100°C.

Aliquots (100µl/well) of the thermally treated fusion were then applied to the plate and incubated for 1 hour, shaken at 37°C. The plate was washed 3x in PBS + Tween 20.

The plate was developed and read as per the functional ELISA described above.

### 5.2 Denaturant stability studies

Denaturant studies were performed as per the functional ELISA above. To test the stability of the SacAK-F3- V_{NAR}, aliquots (200µg/ml) were treated with a range of urea concentrations up to 8M urea for 1 hour at room temperature then diluted to 10µg/ml (giving a maximum urea concentration of 0.4M). The plate was then probed with these aliquots to prevent the bound HEL from being denatured.

### Example 6: Cloning of V_{H}H gene constructs

Generation of the *Sulfolobus acidocaldarius* adenylate kinase (SacAK: GenBank: YP255258) construct was as described in Example 1.

Similar approaches were used for the other thermostable kinases as described herein (SEQ ID NOs 1-7).

In brief, the thermostable kinases genes were re-synthesised with *E*. *coli* codon bias or, if the codon usage was acceptable, the genes of interest were PCR amplified from genomic DNA. Gene constructs were sequence verified and subcloned into the destination vector, as detailed in Example 1. Final expression constructs were also sequence verified.

The HEL specific V_{H}H fragment (GenBank: 1RJC_Acession 29 Oct 2010) was synthesised with a high expressing *E*. *coli* codon bias and a BamHI site at the 5' end, and then inserted into the commercially available pENTR/D-TOPO (Invitrogen). Cloning for the entry vector was carried out using chemically competent TOP10 cells (C404006, Invitrogen, Paisley, UK).

Recombination of the destination vector and entry vector was carried out using LR clonase II mix (11791020, Invitrogen, Paisley, UK) as per the manufacturer's instructions.

The gateway linker region, consisting of the gateway recombination site, was replaced with a flexible linker (F3) with the amino acid repeats [GGGGS]₃. The linker region was created as oligonucleotides (Sigma-Genosys, UK) with overlapping ends corresponding to the cleaved BamHI and SalI restriction sites. The oligonucleotides were annealed by incubating at 90°C prior to insertion through traditional restriction digest of the vector and ligation.

Completed expression vectors were then transformed into *E*. *coli* strain BL21, BLR, RV308 or Origami and stored in cryoprotective beadstocks (PL170, Microbank, Prolab Diagnostics, Canada).

Synthesis and recombination of the constructs was confirmed by sequencing (Beckman Coulter Genomics, Takeley, UK) as was the insertion of alternative linker regions in the fusion protein.

### Example 7: Expression of V_{H}H fusion proteins in E. coli

Expression was achieved using *E*. *coli* RV308 strain in Modified Terrific Broth (T0918) or alternative media (Luria-Bertani -Broth or minimal medium). Primary seed (Luria-Bertani Broth + Tetracycline (T7660) 10 µg/ml) was inoculated with one colony from a Luria-Bertani Agar + Tetracyline (10 µg/ml) plate and left to grow overnight at 200rpm and 37°C. Expression from other *E*. *coli* strains had limited effect on the expression levels produced and subsequent down-stream purification.

After overnight growth the primary seed was inoculated at 5ml/litre into the Modified Terrific Broth flask culture (+ 10 µg/ml Tet); 200rpm 37°C with OD₆₀₀ monitored. When an OD₆₀₀>4 was achieved the cells were harvested by centrifugation at 3000rpm for 30 minutes using a RC3BP (Sorvall, UK) centrifuge with H6000A rotor at 4°C.

The resulting cell paste was then resuspended in PBS and stored as aliquots at - 80°C until needed.

### Example 8: Purification of Sac-F3-VH fusion protein.

Camelid fusion proteins were purified essentially as described above (Example 3). Specifically:

Cell paste was lysed by sonication to break the cells in a high salt buffer (20mM Trizma-Base, 0.9M NaCl, 10mM MgCl pH8.0) with 2mM Glutathione G4251) added. The soluble fraction of the homogenate was then incubated at 80°C for 30 minutes to precipitate out all non-thermostable proteins. Insoluble proteins were removed by centrifugation and the soluble fraction was loaded onto a 5ml Blue Sepharose FF Hitrap column. The glutathione was removed using a 1 hour gradient into the same buffer lacking glutathione. The fusion protein was then eluted using 20mM Trizma-base, 0.9M NaCl, 10mM MgCL, 10mM ATP (A3377) and AMP (A1752) pH8.0. The column was run at 1 ml minute⁻¹, fractions were collected and analysed by SDS-PAGE gel and the fractions containing the final fusion protein product were pooled.

SDS-PAGE gels were run on NuPage 4-12% BisTris 12 well gels with NuPage MES SDS Running Buffer. Gels were developed using Simply Blue Safe Stain.

### Example 9: Detection reagents for viral pathogens; e.g. ebola

Detection reagents are generated essentially as described in Examples 1-3 (shark antibody) or Examples 6-8 (camelid antibody). The single domain antibody is cloned as a BamHI-Xhol fragment into an expression vector, such as the pMTL expression vector described above, downstream of the Sac adenylate kinase gene.

Suitable single domain antibody fragments such as the ebola specific shark Ig_{NAR} domains ("Isolation and characterisation of Ebolavirus-specific recombinant antibody fragments from murine and shark immune libraries. 2011 Goodchild SA, Dooley H, Schoepp RJ, Flajnik M, Lonsdale SG. Mol Immunol. 48:2027-37) may be used to generate specific reagents.

Expression and purification of the sac-F3-IgNAR reagents may be achieved as above. Thermal stability and specificity for the target antigen would be determined essentially as outlined in Examples 4 and 5.

The reagent could be used in a standard detection assay, such as an ELISA. In brief, a capture antibody would be coated onto a solid support and remaining binding sites blocked by addition of a blocking agent such as 5% skimmed milk (3% BSA, 5% gelatin, or 0.1 % Tween 20 could be used either in conjunction with the skimmed milk or in its place). A suitable clinical sample, in this case clarified whole blood or serum, would be applied to the capture-antibody-coated solid support and the target antigen (virus) allowed to bind. Unbound material would be removed by washing and/or selective removal of the solid support from the applied clinical sample.

The reagent can also be used in a magnetic bead-based solid support assay. Essentially the assay is performed as described above except that the capture antibody is coated onto a magnetic bead. The antigen capture and detection reactions are then carried out on beads which may be removed from the clinical sample matrix by attraction to a magnet. Rapid washing may also be achieved by mixing the beads with a saline solution, e.g. phosphate buffered saline containing 0.1% Tween 20.

### Example 10: Detection reagents for bacterial pathogens;

Detection reagents are generated essentially as described in Examples 6-8. A single domain antibody fragment is suitable, such as those derived from library screens of camelid antibodies (e.g "Rugged single domain antibody detection elements for Bacillus anthracis spores and vegetative cells. Walper SA, Anderson GP, Brozozog Lee PA, Glaven RH, Liu JL, Bernstein RD, Zabetakis D, Johnson L, Czarnecki JM, Goldman ER. PLoS One. 2012;7(3):e32801. Epub 2012 Mar 6." Or "Evaluation of a nanobody phage display library constructed from a Brucella-immunised camel. Abbady AQ, Al-Mariri A, Zarkawi M, Al-Assad A, Muyldermans S. Vet Immunol Immunopathol. 2011 Jul 15;142(1-2):49-56. Epub 2011 Apr 14"). The antibody is fused at the C-terminus of the Thermotoga maritima acetate kinase to generate the fusion protein Tma Acetate kinase-F3 linker- V_{H}. The fusion protein is expressed in RV308 or BL21 E. coli strain and the enzyme purified by heat treatment of cell lysate at 80°C for 30 minutes. Optionally the fusion protein may be further purified by binding to a dye-ligand column (e.g. Blue sepharose).

The reagent can then be used in a solid phase binding assay in either an ELISA format or on magnetic beads, essentially as described in example 9. The acetate kinase activity may be detected in a reaction to generate ATP, using highly purified ADP (up to 10mM) and acetyl phosphate (up to 100mM). ATP can be detected by a variety of means including bioluminescent detection using luciferin/luciferase.

A similar approach may be taken to detect the product of a bacterial pathogen, especially protein toxins that may be closely linked with the pathogenesis. Examples of antibodies that may be generated and used in such constructs include "Isolation of a highly thermal stable lama single domain antibody specific for Staphylococcus aureus enterotoxin B. Graef RR, Anderson GP, Doyle KA, Zabetakis D, Sutton FN, Liu JL, Serrano-González J, Goldman ER, Cooper LA. BMC Biotechnol. 2011 Sep 21:11:86" and "Camelid single domain antibodies (VHHs) as neuronal cell intrabody binding agents and inhibitors of Clostridium botulinum neurotoxin (BoNT) proteases. Tremblay JM, Kuo CL, Abeijon C, Sepulveda J, Oyler G, Hu X, Jin MM, Shoemaker CB. Toxicon. 2010 Nov;56(6):990-8. Epub 2010 Jul 14" and "Isolation and Characterization of Clostridium difficile Toxin-Specific Single-Domain Antibodies. Hussack G, Arbabi-Ghahroudi M, Mackenzie CR, Tanha J. Methods Mol Biol. 2012;911:211-39".

### Example 11: Detection and therapeutic agents for metalloprotease.

A significant advantage of single domain antibodies, such as those from shark and camelid is their ability to bind the active site of enzymes, characterised by their deep hydrophobic cleft. Antibodies may be selected for this purpose from single domain antibody libraries, in phage display vectors or equivalent, by altering the binding and elution conditions to favour hydrophobically bound antibodies and/or those eluted by substrate competition. An example of such antibodies, suitable for this purpose is described in " Molecular imprint of enzyme active site by camel nanobodies: rapid and efficient approach to produce abzymes with alliinase activity.Li JW, Xia L, Su Y, Liu H, Xia X, Lu Q, Yang C, Reheman K. J Biol Chem. 2012 Apr 20;287(17):13713-21. Epub 2012 Feb 28".

Such single domain antibodies may be expressed as a fusion with one or more of the thermostable kinases identified herein, such as the uridylate (UMP) kinase from Thermotoga maritima. The fusion is constructed such that the antibody domain is on the N-terminus of the kinase to generate a fusion protein, IgNAR-helical linker-Tma UMP kinase. The reagent is cloned, expressed and purified essentially as described in Examples 1-3, with a high temperature purification step (80°C for 30 minutes) followed by an affinity capture step on Blue Sepharose. Other dye-ligand columns may also optionally be used to purify the fusion protein.

The reagent can be used in a detection or diagnostic assay, e.g. for the New Delhi metalloprotease associated with high level antibiotic resistance to beta-lactamase antibiotics. The assay may be formatted in a similar way to the solid phase binding assays described above using a separate capture antibody to enrich the target from a clinical sample or culture of bacterial pathogen. The UMP-kinase activity may be measured in a reaction that generate ATP, involving the co-substrates ADP (up to 100mM) and UDP (up to 100mM) at near neutral pH. The ATP generated may be detected by a number of means, including bioluminescent detection with luciferin-luciferase.

### Example 12: Multivalent display of single domain antibodies on multimeric thermostable kinases.

The multimeric display of single domain antibodies may be achieved by co-expressing two or more fusion constructs in the same *E. coli* cell. Preferentially a recombination-defective *E*. *coli* strain is used to minimise any chance of rearrangement of the gene constructs during expression. The two fusion protein constructs may be expressed from the same plasmid using the same or different promoters. Alternatively two different compatible plasmids may be used with one gene construct expressed from each. The ratio of different fusion proteins may be varied by using high or low copy number plasmids and/or high, medium or low expressing promoters. Any expressed multimeric thermostable kinase will have a variety of different multimeric-antibody species.

Two constructs are generated such that different single domain antibodies are attached to the thermostable UMP-kinase from *Thermotoga maritima,* enabling hexameric display of the single domain antibodies. The two constructs are expressed from a single pMTL vector essentially as described in Example 1, with each gene construct driven by its own promoter. The constructs are generated such that the fusions are Tma UMP-kinase-F3 linker-IgNAR1 and Tma UMP-kinase-helical linker-IgNAR2. The constructs are expressed at similar levels in *E*. *coli* RV308 (recombinase-negative) and can be expressed and purified essentially as described in Examples 1-3 above. The population of antibody-displaying hexameric kinases will include constructs expressing different ratios of IgNAR1 to IgNAR2 fusions. Optionally constructs may be further purified using an affinity column loaded with the ligands for each of the IgNAR domain to ensure that all hexamers contain at least one of each antibody domain.

A specific example would be the hexameric display of single domain antibodies recognising different epitopes on both *C. difficile* toxin A and toxin B, such that the constructs are Tma UMP-kinase-F3 linker-IgNAR;toxin A and Tma UMP-kinase-F3 linker-IgNAR;toxin B. The equivalent camelid domains such as those described in the Hussack paper (Example 10) would also be valuable for developing this type of reagent.

Similar constructs can be generated with any of the other multimeric thermostable kinases described herein, such that di-, tri-, tetra-, penta-, hexa-, or octa-valent display of single domain antibodies can be achieved.

### Example 13: Applications of mosaic (multivalent) antibody display reagents in detection and diagnostics.

### i) Simple capture assays using mosaic reagents.

The multimeric, "mosaic" antibody-thermostable kinase detection reagents may be used in any of the different assay formats such as those described in the examples above, e.g. for detection of bacteria, toxins, viral pathogens, specific enzymes or other analytes. The avidity effect caused by having multiple copies of one or more antibody domain make them extremely useful antibody detection reagents for high sensitivity and/or rapid detection systems. The ability to co-express the antibody reagent and the detection enzyme (thermostable kinase) also make them simple and easy to use for rapid generation of new reagents.

Preferred applications are those where the different single domain antibodies allow the detection of target species, using a single reagent, where there is significant antigenic diversity that might not allow the use of a single monoclonal antibody. For example, viral pathogens such as norovirus and influenza have the ability to rapidly shift antigen diversity and evade monoclonal antibody binding. By having two or more antibodies recognising different antigens, this risk is minimised. Similarly diseases where two different toxins, either individually or in combination, can mediate disease may be beneficially detected using the reagents described here. This is the case for *Clostridium diffcile* where toxin B either with or without toxin A can mediate disease; both toxins being recognised as risk factors. Many other examples of the benefits of multivalent detection reagents are known to those familiar with the art and this is often cited as one of the benefits of using polyclonal antibody reagents. These are however, slow to make, tend to be variable in quality and do not have the benefits of the highly-defined, enzyme-tagged polyvalent reagents described here.

### A specific example of the use of such an assay:

A polyvalent Tma UMP-kinase was generated as described in Example 12, with two different Ig_{NAR} domains recognising epitopes on *C-difficile* toxin A and B, respectively. Stool extracts from suspected clinical patients were processed using standard methods and applied to an ELISA assay coated with capture reagents (polyclonal antibodies) recognising both *C. difficile* toxins A and B. The plates were incubated and unbound material removed by washing. The UMP-kinase detection reagent was added and the plate incubated at 37°C for 30 minutes to allow binding of the Ig_{NAR} domains to the captured toxin molecules. The plate was again washed to remove unbound detection reagent and the UMP-kinase activity measured by simultaneous addition of ADP, UDP luciferin and luciferase in magnesium acetate buffer. The assay can be used for rapid detection of clinical isolates expressing toxin B and toxins A and B. The assay would also detect toxin A only clinical isolates.

A further example would be use of a mosaic detection reagent recognising norovirus genogroups GI and GII. Multivalent reagents are generated by expressing separate single domain antibodies, specific for GI and GII respectively, fused to the trimeric thermostable adenylate kinase from Sulfolobus acidocaldarius. Expression and purification of the reagents and subsequent use in the assay are as described above.

### ii) recruitment of additional detection reagents.

To increase the sensitivity of the detection reaction, one of the fusion protein generated is specific for an additional thermostable kinase. For example, in an assay to detect anthrax spores, multivalent detection reagents are generated using two constructs; e.g. Tma UMP-kinase-F3-linker- anthrax spore specific V_{H} domain, and Tma UMP-kinase-F3-linker- single domain antibody for Tma acetate kinase. The genes are expressed from the same plasmid but using different regulated promoters such that the fusion proteins are expressed in a ratio of 1 anthrax spore specific fusion to 2 acetate kinase specific fusion. This gives rise to a distribution of hexameric UMP-kinase fusion with a bias towards 2 anthrax spore specific single domain antibodies and 4 acetate kinase single domain antibodies. When used in an assay, using substrates ADP, UDP, acetyl phosphate, followed by luciferin and luciferase, this results in up to 8- fold greater signal intensity compared to the UMP kinase reagent alone (based on the recruitment of 4 additional acetate kinase dimers capable of catalysing the production of ATP).

A similar approach may be used to generate different types of diagnostic signal. For example the co-expression of (i) a fusion protein comprising a trimeric Sac-adenylate kinase fusion with (ii) an antibody for a chlamydial antigen together with an antibody specific for a fluorophore allows for rapid detection in a standard ATP-generating assay, as described above, as well as staining of tissue sections to determine the intracellular fate of the pathogen (i.e. indicating whether infection has been established). A similar approach could be used with a single domain antibody for a tracer molecule which could be imaged in a whole body system, or via histology staining.

## Claims

1. A single-chain fusion protein comprising:
(i) a multimeric thermostable kinase and
(ii) a single-domain antibody, or a fragment of said single-domain antibody that is capable of binding to the same antigen to which the single-domain antibody binds.

2. A fusion protein according to Claim 1, wherein the multimeric thermostable kinase is a trimeric, tetrameric, hexameric or octameric thermostable kinase.

3. A fusion protein according to Claim 1 or 2, wherein the multimeric thermostable kinase is an adenylate kinase, acetate kinase, uridine monophosphate (UMP) kinase, pyruvate kinase, or butyrate kinase.

4. A fusion protein according to any of Claims 1 to 3, wherein the multimeric thermostable kinase is a trimeric adenylate kinase.

5. A fusion protein according to any of Claims 1 to 3, wherein the multimeric thermostable kinase is from a *Sulfolobus* species, such as *S. acidocaldarius.*

6. A fusion protein according to any of Claims 1-5, wherein the multimeric thermostable kinase is a trimeric adenylate kinase from *S. acidocaldarius.*

7. A fusion protein according to any of Claims 1-6, wherein the single-domain antibody is a shark new antigen receptor (IgNAR) or fragment thereof; or wherein the single-domain antibody is a camelid antibody or fragment thereof.

8. A polynucleotide sequence encoding a fusion protein according to any of Claims 1-7.

9. A plasmid comprising a polynucleotide sequence according to Claim 8.

10. A method of producing a fusion protein as defined in any of Claims 1-7, comprising:
(i) expressing the polynucleotide sequence according to Claim 8 or a plasmid according to Claim 9 in a host cell such as *E*. *coli*; and
(ii) purifying the expressed fusion protein from the cytoplasm of the host cell.

11. A method of preparing a single-domain antibody, or a fragment of said single-domain antibody that is capable of binding to the same antigen to which the single-domain antibody binds, the method comprising:
(i) expressing the single-domain antibody or antibody fragment as a single-chain fusion protein with a multimeric thermostable kinase, in a host cell such as *E. coli*; and
(ii) purifying the fusion protein from the cytoplasm of the host cell.

12. A method according to Claim 11, wherein the fusion protein is as defined in any of Claims 1-7.

13. A multimeric fusion protein complex comprising two or more fusion proteins according to any of Claims 1-7.

14. A multimeric fusion protein complex according to Claim 13, wherein the single-domain antibodies or antibody fragments fused to the thermostable kinase monomers of the two or more fusion proteins are the same or different.

15. A reporter molecule for detecting an analyte on or within a sample, the reporter molecule comprising a fusion protein according to any of Claims 1-7 or a multimeric fusion protein complex according to Claim 13 or 14.

16. A detection kit comprising:
(i) a fusion protein according to any of Claims 1-7, a multimeric fusion protein complex according to Claim 13 or 14, or a reporter molecule according to Claim 15, and
(ii) a substrate for the multimeric thermostable kinase.

17. An *in vitro* method of detecting an analyte on or within a sample, comprising:
(i) contacting the sample with a reporter molecule according to Claim 15, wherein the single-domain antibody or antibody fragment binds the analyte to form an antibody-analyte complex; and
(ii) detecting the antibody-analyte complex, such as by detecting the kinase activity of the thermostable kinase.

## Patentansprüche

1. Ein-Ketten-Fusionsprotein, das Folgendes umfasst:
(i) eine multimere thermostabile Kinase und
(ii) einen Ein-Domänen-Antikörper oder ein Fragment des genannten Ein-Domänen-Antikörpers, der sich an dasselbe Antigen binden kann, an das sich der Ein-Domänen-Antikörper bindet.

2. Fusionsprotein nach Anspruch 1, wobei die multimere thermostabile Kinase eine trimere, tetramere, hexamere oder oktamere thermostabile Kinase ist.

3. Fusionsprotein nach Anspruch 1 oder 2, wobei die multimere thermostabile Kinase eine Adenylatkinase, Acetatkinase, UMP-(Uridinmonophosphat)-Kinase, Pyruvatkinsase oder Butyratkinase ist.

4. Fusionsprotein nach einem der Ansprüche 1 bis 3, wobei die multimere thermostabile Kinase eine trimere Adenylatkinase ist.

5. Fusionsprotein nach einem der Ansprüche 1 bis 3, wobei die multimere thermostabile Kinase von einer *Sulfolobus* Spezies wie *S. acidocaldarius* ist.

6. Fusionsprotein nach einem der Ansprüche 1 - 5, wobei die multimere thermostabile Kinase eine trimere Adenylatkinase von *S. acidocaldarius* ist.

7. Fusionsprotein nach einem der Ansprüche 1 - 6, wobei der Ein-Domänen-Antikörper ein igNAR (neuer Hai-Antigen-Rezeptor) oder ein Fragment davon ist; oder wobei der Ein-Domänen-Antikörper ein Camelide-Antikörper oder ein Fragment davon ist.

8. Polynukleotidsequenz, die ein Fusionsprotein nach einem der Ansprüche 1 - 7 kodiert.

9. Plasmid, das eine Polynukleotidsequenz nach Anspruch 8 umfasst.

10. Verfahren zum Produzieren eines Fusionsproteins gemäß Definition in einem der Ansprüche 1 - 7, das Folgendes beinhaltet:
(i) Exprimieren der Polynukleotidsequenz nach Anspruch 8 oder eines Plasmids nach Anspruch 9 in einer Wirtszelle wie *E. coli;* und
(ii) Reinigen des exprimierten Fusionsproteins vom Zytoplasma der Wirtszelle.

11. Verfahren zur Herstellung eines Ein-Domänen-Antikörpers oder eines Fragments des genannten Ein-Domänen-Antikörpers, der sich an dasselbe Antigen binden kann, an das sich der Ein-Domänen-Antikörper bindet, wobei das Verfahren Folgendes beinhaltet:
(i) Exprimieren des Ein-Domänen-Antikörpers oder Antikörperfragments als Ein-Ketten-Fusionsprotein mit einer multimeren thermostabilen Kinase in einer Wirtszelle wie *E. coli*; und
(ii) Reinigen des Fusionsproteins vom Zytoplasma der Wirtszelle.

12. Verfahren nach Anspruch 11, wobei das Fusionsprotein wie in einem der Ansprüche 1-7 definiert ist.

13. Multimerer Fusionsproteinkomplex, der zwei oder mehr Fusionsproteine nach einem der Ansprüche 1 - 7 umfasst.

14. Multimerer Fusionsproteinkomplex nach Anspruch 13, wobei die Ein-Domänen-Antikörper oder Antikörperfragmente, die an die thermostabilen Kinasemonomere der zwei oder mehr Fusionsproteine fusioniert sind, gleich oder unterschiedlich sind.

15. Reporter-Molekül zum Nachweisen eines Analyts auf oder in einer Probe, wobei das Reporter-Molekül ein Fusionsprotein nach einem der Ansprüche 1 -7 oder einen multimeren Fusionsproteinkomplex nach Anspruch 13 oder 14 umfasst.

16. Nachweiskit, der Folgendes umfasst:
(i) ein Fusionsprotein nach einem der Ansprüche 1 - 7, einen multimeren Fusionsproteinkomplex nach Anspruch 13 oder 14 oder ein Reporter-Molekül nach Anspruch 15, und
(ii) ein Substrat für die multimere thermostabile Kinase.

17. *In-vitro*-Verfahren zum Nachweisen eines Analyts auf oder in einer Probe, das Folgendes beinhaltet:
(i) Inkontaktbringen der Probe mit einem Reporter-Molekül nach Anspruch 15, wobei sich der Ein-Domänen-Antikörper oder das Antikörperfragment an das Analyt bindet, um einen Antikörper-Analyt-Komplex zu bilden; und
(ii) Nachweisen des Antikörper-Analyt-Komplexes, zum Beispiel durch Nachweisen der Kinaseaktivität der thermostabilen Kinase.

## Revendications

1. Protéine de fusion monocaténaire comprenant :
(i) une kinase thermostable multimérique et
(ii) un anticorps à un seul domaine, ou un fragment dudit anticorps à un seul domaine qui est capable de se lier au même antigène que celui auquel l'anticorps à un seul domaine se lie.

2. Protéine de fusion selon la revendication 1, dans laquelle la kinase thermostable multimérique est une kinase thermostable trimérique, tétramérique, hexamérique ou octamérique.

3. Protéine de fusion selon la revendication 1 ou 2, dans laquelle la kinase thermostable multimérique est une adénylate kinase, une acétate kinase, une uridine monophosphate (UMP) kinase, une pyruvate kinase, ou une butyrate kinase.

4. Protéine de fusion selon l'une quelconque des revendications 1 à 3, dans laquelle la kinase thermostable multimérique est une adénylate kinase trimérique.

5. Protéine de fusion selon l'une quelconque des revendications 1 à 3, dans laquelle la kinase thermostable multimérique provient d'une espèce *Sulfolobus*, telle que *S*. *acidocaldarius*.

6. Protéine de fusion selon l'une quelconque des revendications 1 à 5, dans laquelle la kinase thermostable multimérique est une adénylate kinase trimérique provenant de *S. acidocaldarius.*

7. Protéine de fusion selon l'une quelconque des revendications 1 à 6, dans laquelle l'anticorps à un seul domaine est un nouveau récepteur d'antigène de requin (IgNAR) ou un fragment de celui-ci ; ou dans laquelle l'anticorps à un seul domaine est un anticorps de camélidé ou un fragment de celui-ci.

8. Séquence polynucléotidique codant pour une protéine de fusion selon l'une quelconque des revendications 1 à 7.

9. Plasmide comprenant une séquence polynucléotidique selon la revendication 8.

10. Procédé de production d'une protéine de fusion telle que définie dans l'une quelconque des revendications 1 à 7, comprenant :
(i) l'expression de la séquence polynucléotidique selon la revendication 8 ou d'un plasmide selon la revendication 9 dans une cellule hôte telle qu'*E. coli* ; et
(ii) la purification de la protéine de fusion exprimée à partir du cytoplasme de la cellule hôte.

11. Procédé de préparation d'un anticorps à un seul domaine, ou d'un fragment dudit anticorps à un seul domaine qui est capable de se lier au même antigène que celui auquel l'anticorps à un seul domaine se lie, le procédé comprenant :
(i) l'expression de l'anticorps à un seul domaine ou du fragment d'anticorps sous la forme d'une protéine de fusion monocaténaire comprenant une kinase thermostable multimérique, dans une cellule hôte telle qu'*E. coli* ; et
(ii) la purification de la protéine de fusion à partir du cytoplasme de la cellule hôte.

12. Procédé selon la revendication 11, dans lequel la protéine de fusion est telle que définie dans l'une quelconque des revendications 1 à 7.

13. Complexe de protéines de fusion multimériques comprenant deux ou plusieurs protéines de fusion selon l'une quelconque des revendications 1 à 7.

14. Complexe de protéines de fusion multimériques selon la revendication 13, dans lequel les anticorps à un seul domaine ou les fragments d'anticorps fusionnés aux monomères kinases thermostables desdites deux protéines de fusion ou plus sont identiques ou différents.

15. Molécule reporter destinée à la détection d'un analyte sur ou dans un échantillon, la molécule reporter comprenant une protéine de fusion selon l'une quelconque des revendications 1 à 7 ou un complexe de protéines de fusion multimériques selon la revendication 13 ou 14.

16. Trousse de détection comprenant :
(i) une protéine de fusion selon l'une quelconque des revendications 1 à 7, un complexe de protéines de fusion multimériques selon la revendication 13 ou 14, ou une molécule reporter selon la revendication 15, et
(ii) un substrat pour la kinase thermostable multimérique.

17. Procédé *in vitro* de détection d'un analyte sur ou dans un échantillon, comprenant :
(i) la mise en contact de l'échantillon avec une molécule reporter selon la revendication 15, l'anticorps à un seul domaine ou le fragment d'anticorps se liant à l'analyte pour former un complexe anticorps-analyte ; et
(ii) la détection du complexe anticorps-analyte, par exemple en détectant l'activité de kinase de la kinase thermostable.
